# EUROPEAN PATENT SPECIFICATION

(11) **EP 2 364 163 B1**
(45) Date of publication and mention of the grant of the patent: **16.03.2016**
(21) Application number: 09777271.9
(22) Date of filing: 17.07.2009
(51) Int. Cl.: A61K 38/18

(54) **COMPOSITION COMPRISING IN VITRO EXPANDED T-LYMPHOCYTES AND VESSEL FORMATION INHIBITORS SUITABLE IN THE TREATMENT OF CANCER**
ZUSAMMENSETZUNG MIT IN-VITRO EXPANDIERTEN T-LYMPHOZYTEN UND GEFÄSSBILDUNGSHEMMERN ZUR BEHANDLUNG VON KREBS
COMPOSITION COMPRENANT DES LYMPHOCYTES T EXPANSÉS IN VITRO ET INHIBITEURS DE LA FORMATION DE VAISSEAUX APPROPRIÉS DANS LE TRAITEMENT D'UN CANCER

(30) Priority: 18.07.2008 US 81804 P; 18.07.2008 DK 200801025
(43) Date of publication of application: 14.09.2011
(73) Proprietor: SentoClone International AB, 171 65 Solna (SE)
(72) Inventor: WINQVIST, Ola, S-756 53 Uppsala (SE); THÖRN, Magnus, S-752 26 Uppsala (SE)
(74) Representative: Schöneborn, Holger
(86) International application number: PCT/EP2009/005216
(87) International publication number: WO 2010/006808

(56) References cited:
- WO-A-2007/071388
- WO-A-2007/071409
- KJELL DAHL ET AL: "Metinel Node-The First Lymph Node Draining a Metastasis-Contains Tumor-Reactive Lymphocytes" ANNALS OF SURGICAL ONCOLOGY, SPRINGER-VERLAG, NE, vol. 15, no. 5, 26 February 2008 (2008-02-26), pages 1454-1463, XP019598064 ISSN: 1534-4681
- MANNING ELIZABETH A ET AL: "A vascular endothelial growth factor receptor-2 inhibitor enhances antitumor immunity through an immune-based mechanism." CLINICAL CANCER RESEARCH : AN OFFICIAL JOURNAL OF THE AMERICAN ASSOCIATION FOR CANCER RESEARCH 1 JUL 2007, vol. 13, no. 13, 1 July 2007 (2007-07-01), pages 3951-3959, XP002550185 ISSN: 1078-0432
- LI BETTY ET AL: "Vascular endothelial growth factor blockade reduces intratumoral regulatory T cells and enhances the efficacy of a GM-CSF-secreting cancer immunotherapy." CLINICAL CANCER RESEARCH : AN OFFICIAL JOURNAL OF THE AMERICAN ASSOCIATION FOR CANCER RESEARCH 15 NOV 2006, vol. 12, no. 22, 15 November 2006 (2006-11-15), pages 6808-6816, XP002550186 ISSN: 1078-0432
- VEERAVAGU ANAND ET AL: "Vascular endothelial growth factor and vascular endothelial growth factor receptor inhibitors as anti-angiogenic agents in cancer therapy." RECENT PATENTS ON ANTI-CANCER DRUG DISCOVERY JAN 2007, vol. 2, no. 1, January 2007 (2007-01), pages 59-71, XP002550184 ISSN: 1574-8928
- GLADE-BENDER J ET AL: "VEGF BLOCKING THERAPY IN THE TREATMENT OF CANCER" EXPERT OPINION ON BIOLOGICAL THERAPY, ASHLEY, LONDON, GB, vol. 3, no. 2, 1 April 2003 (2003-04-01), pages 263-276, XP009060849 ISSN: 1471-2598

## Description

### Field of the invention

The present invention relates to an improved therapy comprising inhibitors of vessel formation inhibitors, notably, inhibitors of VEGF, in combination with tumour-reactive T-lymphocytes obtained by a *in vitro* method for expansion and activation of tumour-reactive lymphocytes, in particular CD4+ helper and/or CD8+ T-lymphocytes. The tumour-reactive T-lymphocytes are not CD4+ CD25+^{Hi} lymphocytes, i.e. the present invention does not cover regulatory T-lymphocytes. Furthermore, the tumour-reactive T-lymphocytes used in present invention are different from so called tumour infiltrating lymphocytes (TILs). The tumour-reactive T-lymphocytes used in the present invention does are so-called SNALs (Sentinel node Acquired Lymphocytes) or MNALs (Metinel node Acquired Lymphocytes). The invention also relates to a composition comprising inhibitors of vessel formation inhibitors, notably inhibitors of VEGF, and tumour-reactive T-lymphocytes. Normally, in order to allow a suitable treatment regimen with flexibility relating to dose, administration route and administration time, the composition comprises two separate preparations, one containing one or more inhibitors of vessel formation inhibitors and another one containing tumour-reactive T-lymphocytes. The tumour-reactive T-lymphocytes can be obtained by an *in vitro* method for expansion and activation of tumour-reactive lymphocytes, in particular CD4+ helper and/or CD8+ T-lymphocytes.

### Background of the invention

According to the immune surveillance hypothesis, the immune system is continuously sensitized against developing tumours, where experimental evidence strongly supports this notion. The identification of specific tumour antigens has created new possibilities for tumour immunotherapy and many immunotherapeutic approaches are now being translated into clinical trials. Among these, adoptive transfer of tumour antigen-specific lymphocytes seems particularly promising. These attempts have, so far, usually been based on either mononuclear cells from peripheral blood or tumour infiltrating lymphocytes (TIL) separated from fresh tumour specimens. In recent trials, treatment of patients with malignant melanoma with autologous transfer of expanded TILs, objective response rates of up to 51% has been reported. TIL cells are few, they are frequently unresponsive (anergic) due to immunosuppressive mechanisms from the tumour creating long periods for expansions to occur (several months). Furthermore, the protocols have been aiming towards the expansion of CD8⁺ cytotoxic T-cells and the cells have been reintroduced into patients preconditioned with chemotherapy and in addition the patients have been treated with high doses of interleukin-2 to provide survival of CD8⁺ T-cells.

Angiogenesis, also called neovascularization, is a fundamental process whereby new blood vessels are formed. Under normal physiological conditions angiogenesis is highly regulated and essential for reproduction, embryonic development, tissue repair and wound healing. However, angiogenesis also occurs under various pathological conditions, including tumour growth and metastasis, inflammatory disorders such as rheumatoid arthritis, psoriasis, osteoarthritis, inflammatory bowel disease, Crohn's disease, ulcerative colitis and various ocular disorders in connection with ocular neovascularization. In fact, angiogenesis occurs in response to various proangiogenic stimuli like growth factors, cytokines and other physiological molecules as well as other factors like hypoxia and low pH.

The angiogenic cascade for development of new blood vessels requires the cooperation of a variety of molecules that regulate necessary cellular processes such as extracellular matrix (ECM) remodelling, invasion, migration, proliferation, differentiation and tube formation. After an initiation phase proangiogenic molecules like VEGF, bFGF, PDGF and others activate the endothelial cells via stimulation of their cell surface receptors (for example VEGFR1/Flt-1 and VEGFR2/Flk-1/KDR). These activated cells undergo a process of cellular proliferation, elevated expression of cell adhesion molecules, increased secretion of proteolytic enzymes and increased cellular migration and invasion. A number of distinct molecules are involved to promote proliferation and invasion, including members of integrin, selectin and immunoglobulin gene super family for adhesion as well as proteolytic enzymes such as matrix metalloproteinase and serine proteinases for degrading the extracellular matrix. Finally, a complex cascade of biochemical signals derived from cell surface receptors interacting with extracellular matrix components and soluble factors, leading to lumen formation and differentiation into mature blood vessels.

Due to the inherent connection of VEGF with various disorders as mentioned above, VEGF has been considered as an appealing target for anticancer therapeutics, especially in combination with conventional chemotherapy, radiotherapy and other antiangiogenic agents. A number of angiogenic inhibitors (VEGF and VEGF-related inhibitors) are known in the art. Mentioning just a few examples; VEGF inhibitory aptamers; e.g. Macugen® (pegaptanib, Pfizer), antibodies or fragments thereof; e.g. anti-VEGF antibodies such as Avastin® (bevacizumab, Genentec), or fragments thereof e.g. Lucentis® (ranibizumab, Genentec) have occurred on the market.

However, as pointed out above, the use of for example Avastin^{®} requires a therapy in combination with cytostatic compounds to have any therapeutic effect. Furthermore, the use of for example Avastin^{®}, have revealed a number of unwanted side-effects, when used as a combination therapy. The main side effects are hypertension and heightened risk of bleeding. Bowel perforation has been reported. Brain capillary leak syndrome, nasal septum perforation, and renal thrombotic microangiopathy have also been reported

### Disclosure of the invention

By use of tumour-reactive T-lymphocytes e.g. obtained by the method disclosed herein in accordance with WO 2004/032951 and WO 2007/071388 (to the same applicant), in combination with one or more inhibitors of VEGF in the treatment of cancer, it is envisaged that cancer can be treated in an efficient manner. Notably, it is envisaged that an improved treatment is obtained. Thus, it is envisaged that an improved treatment is obtained compared to a treatment using either immunotherapy as described herein or an inhibitor of VEGF. By creating a block of angiogenesis by use of an inhibitor of VEGF there will be an increased lack of oxygen, i.e. anoxia, resulting in necrosis of cancer cells that will be taken up and cause an increased presentation and activation of the immune system. Accordingly, immunotherapy in combination with an inhibitor of VEGF seems to supplement each other. One or more of the following beneficial effects may be obtained:
a) an improvement of the overall survival (preferably, compared with treatment of tumour-reactive T-lymphocytes without one or more inhibitors of VEGF or, alternatively, compared with treatment with the same inhibitor of VEGF and a cytostatic compound usually employed in the treatment of the specific type of cancer), i.e. an improvement of at least 10% such as, e.g. at least about 20%, at least about 30%, at least about 40% at least about 50%, at least about 60%, at least about 70% or at least about 80%;
b) a decrease of tumour size (in case of solid tumours); the tumour size can be measured by CT scan, MRI or ultrasound using RECIST criteria and the decrease is at least 10% such as, e.g. at least about 20%, at least about 30%, at least about 40% at least about 50%, at least about 60%, at least about 70% or at least about 80%;
c) a regression of metastases e.g. as measured by CT scan using RECIST criteria; the regression should be at least 10% such as, e.g. at least about 20%, at least about 30%, at least about 40% at least about 50%, at least about 60%, at least about 70% or at least about 80%;
d) a reduction of frequency of harmful side-effects
e) a reduction of the magnitude of side-effects
f) a reduction of the dosage of tumour-reactive T-lymphocytes and/or the dosage of one or more inhibitors of VEGF (compared the dosage of tumour-reactive T-lymphocytes in a with treatment of tumour-reactive T-lymphocytes without one or more inhibitors of VEGF or, alternatively, compared with treatment with the same inhibitor of VEGF and a cytostatic compound usually employed in the treatment of the specific type of cancer), i.e. a dosage reduction of at least 10% such as, e.g. at least about 20%, at least about 30%, at least about 40% at least about 50%, at least about 60%, at least about 70% or at least about 80% of the tumour-reactive T-lymphocytes and/or the one or more inhibitors of VEGF.

It is to be understood that the relevant T-lymphocytes according to present invention are derived from sentinel lymph nodes (SEALs) and/or metinel lymph nodes (MEALs). Such a concept is described by Kjell Dahl et al.: "Metinel node - the first node draining a metastasis - contains tumor-reactive lymphocytes", Annals of Surgical Oncology 2008; 15(5): 1454-1463.

Thus, present invention relates to a composition according to
claim 1.

As mentioned above, such a composition normally comprises two separate preparations each containing one of the active principles. Moreover, the tumour-reactive CD4+ T helper and/or CD8+ T-lymphocytes used are different from tumour infiltrating lymphocytes (TILs).

Moreover, an additive or synergistic effect of the combination compared with administration of the components alone in the same doses/amounts is expected.

The combination therapy is especially suitable in those cases where tumour-reactive cells can be derived from a sentinel or metinel lymph node. In such cases, the tumour-reactive cells can be derived from the same patients, i.e. the cells are autologous. However, it cannot be excluded that allogeneic transplantation in some cases may give an efficient treatment.

### Definitions

By the term "tumour-reactive T-lymphocytes" is intended to mean T-lymphocytes carrying a T cell receptor specific for and recognizing a tumour antigen.

By the term "T helper cells" is intended to mean T-lymphocytes that promote adaptive immune responses when activated.

By the term "Th1 cells" is intended to mean T helper cells that promote cell mediated immune responses when activated, using cytokines such as IFN-gamma.

By the term "Th2 cells" is intended to mean T helper cells promoting humoral immune responses when activated, using cytokines such as IL-4.

By the term "CD4+ helper T-lymphocytes" is intended to mean T-lymphocytes that express CD4; i.e. they are CD4+ T-lymphocytes which are not regarded as regulatory T cells with markers such as high CD25 expression and expression of FOXP3.

By the term "CD8+ T-lymphocytes" is intended to mean T-lymphocytes that express CD8.

By the term "regulatory T-lymphocyte" is intended to mean T-lymphocytes that suppress adaptive immune responses, expressing the transcription factor FoxP3.

By the term "specific activation" of T-lymphocytes is intended to mean antigen specific and MHC restricted T-cell receptor mediated activation. In contrast the term "unspecific activation" of T-lymphocytes is intended to mean a general activation of all T-cells, regardless of T-cell receptor specificity.

By the term "Tumour Infiltrating Lymphocyte" or "TIL" is intended to mean a white blood cell (leukocyte) that has left the blood stream and migrated into a tumour. Therapeutic TILs is usually a preparation of cells, consisting of autologous TILs, that are manipulated in vitro and, upon administration in vivo, re-infiltrate the tumour to initiate tumour cell lysis. Therapeutic TILs are isolated from tumour tissue. Thus, it is the location of the lymphocytes that define TILs. By definition, a TIL is a lymphocyte that has been harvested in tumour tissue. However, functionally they are frequently different since they have been subjected to tumour induced suppression and for example carry epigenetic markers indicating shutdown of functional antitumoral responses, as we have recently discovered. (P Jansson, J immunol, 2008)].

The term "tumour-derived antigen" intends to cover tumour cells, a homogenate of a tumour, which homogenate may be denatured, or tumour proteins, polypeptides or peptides, e.g. in the form of purified, natural, synthetic and/or recombinant protein, polypeptide or peptide. The tumour-derived antigen may be intact molecules, fragments thereof or multimers or aggregates of intact molecules and/or fragments. Examples of suitable polypeptides and peptides are such that comprises from about 5 to about 30 or from about 1-1000 amino acids, such as, e.g. from about 10 to 25 amino acids, from about 10 to 20 amino acids or from about 12 to 18 amino acids or such as, e.g. from about 30-50 amino acids, from about 70-100 amino acids, from about 200-500 amino acids or from about 500-1000 amino acids.. If peptides are used (e.g. in an analogous method to the method described in Reference Example 1 herein), a final molar concentration in the culture is normally from about 0.1 to about 5.0 µM, such as, e.g., from about 0.1 to about 4.0 µM, from about 0.2 to about 3.0 µM, from about 0.3 to about 2.0 µM or from about 0.3 to about 1.0 µM may be used. The tumour-derived antigen may be autologous or heterologous, i.e. arise from the patient to be treated or be obtained from another subject suffering from cancer. In the present Examples the inventors uses an autologous denatured tumour extract, however, as mentioned above, other sources of the tumour-derived antigen may also be feasible for use according to the invention.

By the term "sentinel lymph node" is intended to mean the first lymph node(s) to receive lymphatic drainage from a tumour. The term "metinel lymph node" refers to the first lymph node(s) to receive lymphatic drainage from a metastasis.

By the term "reduction in frequency of side-effects" is intended to mean that harmful side-effects observed in clinical trials using treatment with the combination are less frequent than if treatment was carried out using components alone.

By the term "harmful side-effect" is intended to mean a response to a drug which is noxious and unintended, and which occurs at doses normally used in man for the prophylaxis, diagnosis, or therapy of disease, or for the modification of physiological function.

By the term "reduction in magnitude of side effects" is intended to mean that the measured magnitude and/or frequency of any measurable side effect is reduced.

As mentioned above, administration of the combination may lead to an additive or synergistic effect. An additive effect is typically present if the effect obtained corresponds to "the sum" of effects obtained if the combination was administered alone, whereas a synergistic effect is present if the effect obtained is greater than "the sum" of effects obtained if the combination was administered alone. Both situations are advantageous in that it may be possible to obtain a sufficient effect using a lower amount of the components.

By the term "monoclonal antibodies" is intended to mean monospecific antibodies that are identical being produced by one type of immune cell that are all clones of a single parent cell. Within this definition is also intended to mean fragments, portions or form of antibody that retains the relevant immunoreactivity with VEGF or its receptor.

By the term "epitope", also know as antigenic determinant, is intended to mean the part of a macromolecule that is recognized by the immune system.

By the term "polyclonal antibodies" is intended to mean antibodies that are derived from different B cell lines. They are a mixture of immunoglobulin molecules secreted against a specific antigen, each recognising a different epitope.

By the term "VEGF" is intended to mean the class of proteins classified as Vascular Endothelial Growth Factor proteins. Within this definition is also intended to mean any subtype of, isoform or epitopes of VEGF.

By the term "VEGF inhibitor", "antiangiogenic compound", "angiogenic inhibitor" used interchangeably, is intended to mean compound or compounds that inhibit the activity or production of vascular endothelial growth factors (VEGFs). It refers for example to a protein, such as a monoclonal antibody, antibodies, fragments of antibodies or peptides (cyclic or no-cyclic), nucleic acids, siRNA, ribozymes that inhibit VEGF expression at the nucleic acid level and a small molecules that inhibit VEGF. The term is also intended to mean inhibitors of all isoforms, subtypes and epitopes of VEGF

By the term "pharmaceutical excipient" or "pharmaceutically acceptable excipient", used interchangeably, is intended to mean any therapeutically inactive substance used as a carrier for the active ingredients of a medication.

By the term "relevant T-lymphocytes" is intended to mean tumour-reactive CD4+ helper and/or CD8+ T-lymphocytes that are specific for the cancer type that is to be targeted.

By the term "composition" is intended to mean one or more separate entities or preparations that either alone or in any combination make up the composition. Hence, the term is used to denote either the relevant T-lymphocytes, one or more VEGF-inhibitors and optionally a chemotherapeutic compound each in separate preparations; or the relevant T-lymphocytes in one separate preparation and one or more VEGF-inhibitors and optionally a chemotherapeutic compound in another or in other separate preparations. The term may also used to denote one single composition of the relevant T-lymphocytes, VEGF-inhibitors and optionally a chemotherapeutic compound, provided that all active principles are to be administered at the same point in time and via the same administration route.

In the following, the term "immunotherapy component" is used to denote the T-lymphocyte component and the "antiangiogenic component" is used to denote the component containing one or more VEGF inhibitors, i.e. the two mandatory components in a composition of the invention.

### Immunotherapy component - tumour-reactive T-lymphocytes

The present inventors have previously shown that activation of naive T cells may occur within the highly specialized microenvironment of secondary lymphoid organs, such as the sentinel lymph node. In other words, the sentinel node may be regarded as the primary site for the immune system to encounter tumour antigens.

The inventors have previously disclosed a general method for expansion of tumour-reactive T-lymphocytes from sentinel lymph nodes, showing that it is possible to culture T-lymphocytes obtained from sentinel lymph nodes in order to obtain a culture of tumour-reactive T-lymphocytes (WO 2004/032951 and WO 2007/071388, which is hereby included by reference). The tumour-reactive T-lymphocytes may be used for treating cancer by administering an effective amount of tumour-reactive T-lymphocytes to the patient from which the sentinel nodes were removed.

The relevant T-lymphocytes are obtained by a method for expansion of tumour-reactive CD4+ helper and/or CD8+ T-lymphocytes, wherein specific culturing conditions have been determined and optimized, and wherein specific markers on the T-lymphocytes and in the culture medium are monitored throughout the expansion phase, in order to obtain high numbers of tumour-reactive T-lymphocytes in the shortest possible time span. Furthermore, the invention at the same time provides a method for directing the development of tumour-reactive CD4+ helper and/or CD8+ T-lymphocytes towards specific subpopulations. The T-lymphocytes are not CD4+ CD25+^{Hi} lymphocytes, i.e. the present invention does not cover regulatory T-lymphocytes.

CD4⁺CD25^{Hi} T lymphocytes expressing the transcription factor FoxP3 are considered regulatory T cells (Treg). Tregs have the property to regulate T helper and T cytotoxic cells by inhibiting activation and proliferation and in addition Treg inhibit the production and release of useful Th1 cytokines such as IFN-gamma. Thus, the method according to the present invention promotes the expansion of Thelper cells and T cytotoxic T cells and to avoid expansion of Treg cells.

Activated, proliferating CD4+ helper T-lymphocytes can differentiate into two major subtypes of cells, Th1 and Th2 cells, which are defined on the basis of specific cytokines produced. Th1 cells produce interferon-gamma and interleukin 12 (IL-12), while Th2 cells produce interleukin-4, interleukin-5 and interleukin-13. Th1 T-lymphocytes are believed to promote activation of cytotoxic T lymphocytes (Tc), NK cells, macrophages, and monocytes, all of which can attack cancer cells and generally defend against tumours.

T-helper (CD4+) lymphocytes of type Th1 and Th2 can differentiate into memory cells and effector cells. Memory T-helper (CD4+) lymphocytes are specific to the antigen they first encountered and can be called upon during a secondary immune response, calling forth a more rapid and larger response to the tumour-antigens. There is evidence in humans that lymphocytes survive at least 20 years; perhaps for life. Effector CD4+ T-lymphocytes are active cells producing cytokines and INF-gamma.

Accordingly, encompassed by the present invention are the use of CD4+ helper T-lymphocytes including the subtypes Th1 and Th2 and memory and effector cells derived therefrom, and CD8+ T-lymphocytes.

The tumour-reactive T-lymphocytes most often generated by the method described in Reference Example are CD4+ helper T-lymphocytes. One of the objects of the expansion method is in some respect to imitate the natural pathway of the patient's own immune system, and to a certain degree let the components of the patients immune system determine whether, in the first place, CD4+ helper or CD8+ T-lymphocytes are generated, depending on whether antigen is presented by MHC I or MHC II. In most cases, the antigens will be presented by the class II MHC molecule leading to generation of CD4+ helper T-lymphocytes. However, in some cases CD8+ T-lymphocytes are generated. If CD4+ helper T-lymphocytes are generated, they will be further expanded as described herein; however, the method may also be used for expanding CD8+ cells. The inventors have previously presented detailed methods for the expansion of relevant T-lymphocytes (WO2007/071388 and WO2004/032951), incorporated herein by reference. However, other methods may also be used provided that CD4+ helper T-lymphocytes and/or CD8+ T-lympocytes are generated without significant content of Treg cells, i.e. at the most 1% of the cells may be Treg cells, preferably none of the cells are Treg cells.

Th2 cells produce cytokines that stimulate B cells. This will not result in tumour shrinkage and, accordingly, a pronounced appearance of Th2 cells should be avoided. Instead Th1 cells promote CD8 cytotoxic cells and thus will destroy tumour cells. In order to substantially avoid the generation of tumour-reactive T-lymphocytes of the Th2 type, expansion may be carried out with the addition of one or more substances capable of antagonizing development of Th2 type T-lymphocytes. Examples of such substances are substances capable of neutralizing the interleukins IL-4, IL-5, IL-10, and/or TGF-beta (the latter not being an interleukin) all four of which are required for the establishment of the Th2 cytokine profile and for down regulation of Th1 cytokine production.

Examples of such substances include proteins, polypeptides, peptides, soluble receptors, antibodies, affibodies, and fragments thereof, fusion proteins, synthetic and/or organic molecules, such as, e.g., small molecules, and natural tigands. In a specific embodiment the substances are selected from antibodies that binds to the interleukins, thereby neutralizing them, such as, e.g. anti IL-4 antibody, anti IL-5 antibody and/or anti IL-10 antibody, together with soluble receptors (such as, e.g. TGF-beta receptor I and II) and binding proteins for TGF-beta (such as, e.g. LAP and/or LTBP).

The one or more substances capable of antagonizing development of Th2 type T-lymphocytes, such as, e.g., one or more substances capable of neutralizing IL-4, IL-5, IL-10 and/or TGF-beta may be added on day 1 of the second phase ii) (see Reference Example 1 herein). However, as antibodies are expensive, the addition of antibodies can also be performed in a subsequent step after addition of the substance capable of up-regulating IL-12R on the T-lymphocytes, such as, e.g., one day, two days or three days after addition of the substance capable of up-regulating IL-12R on the T-lymphocytes.

The neutralizing substances should be added in an amount sufficient to neutralize the interleukins, such as, e.g., in a 10-100 fold (molar) excess of the amount of interleukin to be neutralized. When using antibodies, a final concentration of from about 2 to about 4 ng/ml culture medium will normally be needed. For other types of neutralizing substances, a final concentration, giving the same effect as the concentration mentioned for antibodies, should be used.

In order to maintain the suppression of the development of Th2 type T-lymphocytes a further amount of the one or more substance capable of antagonizing development of Th2 type T-lymphocytes, such as, e.g., one or more substance capable of neutralizing IL-4, IL-5, IL-10 and/or TGF-beta may be added regularly the expansion, such as, e.g. every 2^{nd}, 3^{rd} or 4^{th} day of phase ii). It is to be understood that by the term every 2^{nd}, 3^{rd} or 4^{th} is intended to mean that at least one substance capable of antagonizing development of Th2 type T-lymphocytes is added throughout the expansion every 2^{nd}, 3^{rd} or 4^{th} day, starting at the 2^{nd}, 3^{rd} or 4^{th} day after the first addition of the at least one substance capable of antagonizing development of Th2 type T-lymphocytes.

In order to favour the generation of Th1 type tumour-reactive T-lymphocytes, the second phase ii) (see Reference Example 1 herein) may comprise adding one or more substances promoting the development of Th1 type T-lymphocytes. Examples of such substances are substances having agonistic activity towards the IL-7, IL-12, IL-15 and/or IL-21 receptor. More specific, the substances may be agonists for the IL-7, IL-12, IL-15 and/or IL-21 receptor. Examples of such agonists include proteins, polypeptides, peptides, antibodies, affibodies, and fragments thereof, fusion proteins, synthetic and/or organic molecules, such as, e.g., small molecules, and natural ligands. In a specific embodiment the substances are the natural ligands of the IL-7, IL-12, IL-15 and/or IL-21 receptor, respectively, such as IL-7, IL-12, IL-15 and/or IL-21.

The effect of IL-12 is activating the IFN-gamma inducing STAT pathway by stimulating the IL-12R thereby promoting activation of Th1 lymphocytes. The function of IL-21 is to enhance proliferation, activation and development towards a Th1 type of T-lymphocytes.

Both IL-7 and IL-15 work by promoting homeostatic expansion of the T-lymphocytes, enhancing the enumeration of activated Th1 programmed T-lymphocytes.

The expansion method used to provide the relevant T-lymphocytes is preferably one that provides CD4+ tumour-reactive T-lymphocytes of the Th1 type. One further aspect of the invention is that by using the method described herein for expanding tumour-reactive T-lymphocytes, a relatively high amount of T-lymphocytes of the memory type will be obtained. In treating cancer it is of course important that the patient to be treated receive a high amount of effector tumour-reactive CD4+ T-lymphocytes, as these - as mentioned above - promote activation of cytotoxic T lymphocytes (Tc), NK cells, macrophages, and monocytes, all of which can attack cancer cells and generally defend against tumours.

Thus, the relevant T-lymphocytes (CD4+ T helper and/or CD8+ reactive T-lymphocytes) according to present invention may be obtained by stimulating tumour-reactive CD4+ T helper and/or CD8+ T-lymphocytes with tumour-derived antigen together with at least one substance having agonistic activity towards the IL-2 receptor to promote survival of tumour-reactive CD4+ T helper and/or CD8+ T-lymphocytes; and activating and promoting growth of tumour-reactive CD4+ T helper and/or CD8+ T-lymphocytes, wherein the second phase ii) is initiated when the CD25 cell surface marker (or IL-2R marker) is down-regulated on CD4+ T helper and/or CD8+ T-lymphocytes.

By administering a substantial amount of memory tumour-reactive CD4+ T-lymphocytes at the same time, the patient achieve up to life long protection towards recurrence of the tumour or metastasis of the primary tumour.

As it appears from Reference Example 1 herein, the expression of the cell surface activation markers CD25 and CD69 on the T-lymphocytes may be used for determining when to initiate important steps of the present method, such as, e.g., when to initiate the second phase ii). Accordingly, it may be beneficial to continuously monitor the expression of CD25 and CD69 throughout phase i) and phase ii), such as, e.g., every 2^{nd}, every 3^{rd} or every 4^{th} day. However, besides the expansion method disclosed herein, other methods may also be suitable. One advantage with the method described in the Reference Example 1 is the high yield of cells and the possibility of directing the process to provide the T-lymphocytes of choice; however, there may be situations where e.g. a lower yield is acceptable and then other methods may also be used.

As one of the purposes of the present invention is to obtain a composition comprising specific CD4+ tumour-reactive T-lymphocytes, which may be used for administering to a patient, the tumour-reactive T-lymphocytes may be harvested at some point, leading to the termination of the expansion step. The optimal point of time to harvest the tumour-reactive T-lymphocytes is when the expression of CD25 on the T-lymphocytes is down-regulated, where the down-regulation is defined as that 5% or less of the CD4+ T- lymphocyte and/or CD8+ T-lymphocyte population expresses CD25. The optimal point in time to harvest may also be determined based on measurement of the amount of IFN-gamma produced. The IFN-gamma production should be at least 2 fold increased, such as, e.g., at least 3 fold, at least 4 fold or at lest 5 fold increased as compared to initial IFN-gamma production, which normally correspond to a level of IFN-gamma of at least 100 pg/ml of culture medium.

The success of a cancer treatment comprising administration of tumour-reactive T-lymphocytes are determined by factors such as, e.g., the amount of tumour-reactive T-lymphocytes obtained after expanding cells from a sentinel node, i.e. the amount of tumour-reactive T-lymphocytes available for infusion to the patient, the time required to obtain an effective amount of tumour-reactive T-lymphocytes and the concentration and ratio of specific subpopulations of tumour-reactive T-lymphocytes obtained by the expansion method used. All these success factors are envisaged to be of importance also when the treatment is combined with one or more inhibitors of VEGF.

All details described in this paragraph under the heading "Tumour-reactive T-lymphocytes" are also valid for T-lymphocytes contained in a composition containing an immunotherapy component and an angiogenesis component.

### Antiangiogenic component - vessel formation inhibitors, notably Inhibitors of VEGF

Vascular endothelial growth factor (VEGF) is a sub-family of growth factors, more specifically of platelet-derived growth factor family of cysteine-knot growth factors. They are important signaling proteins involved in both vasculogenesis (the *de novo* formation of the embryonic circulatory system) and angiogenesis (the growth of blood vessels from pre-existing vasculature). The most important member is VEGF-A. Other members are Placenta growth factor (PIGF), VEGF-B, VEGF-C and VEGF-D. A number of VEGF-related proteins have also been discovered encoded by viruses (VEGF-E) and in the venom of some snakes (VEGF-F).

Due to the inherent character of VEGF, this class of proteins is of great interest in the treatment of cancer, and has been the target of interest in the therapy using proteins, monoclonal antibodies or anti-body derivatives and small molecules as inhibitors to VEGF or tyrosine kinases stimulated by VEGF. Examples of monoclonal antibodies or antibody derivatives are bevacizumab (Avastin^{®}, used for medical indications in metastatic colorectal cancer, non-small cell lung cancer and metastatic breast cancer) and ranibizumab (Lucentis^{®}). Examples of small molecule inhibitors are sunitinib (Sutent^{®}), sorafenib (Nexavar^{®}), N-Methyl-2-[[3-[(E)-2-pyridin-2-ylethenyl]-1H-indazol-6-yl]sulfanyl]benzamide (Axitinib^{®}), , and 5-[[4-[(2,3-Dimethyl-2H-indazol-6-yl)(methyl)amino]pyrimidin-2-yl]amino]-2-methylbenzenesulfonamide (Pazopanib^{®}).

However in the case of bevacizumab (Avastin^{®}), it has been found that this therapy has to be combined with standard chemotherapy, such as 5-fluorouracil-based chemotherapy or paclitaxel, in order to have any therapeutic effect. In addition to the requirement of combination therapy a number of side-effects have been discovered during Avastin-therapy such as gastrointestinal (GI) perforation, wound healing complications and hemorrhage.

As described above the role of VEGF-inhibitors is mainly to inhibit angiogenesis in tumoural tissues and thereby stop tumour growth. Vascular endothelial growth factor (VEGF)-mediated angiogenesis is thought to play a critical role in tumor growth and metastasis. Consequently, anti-VEGF therapies are being actively investigated as potential anti-cancer treatments, either as alternatives or adjuncts to conventional chemo or radiation therapy. Among the techniques used to block the VEGF pathway are: 1) neutralizing monoclonal antibodies against VEGF or its receptor, 2) small molecule tyrosine kinase inhibitors of VEGF receptors, 3) soluble VEGF receptors which act as decoy receptors for VEGF, and 4) ribozymes which specifically target VEGF mRNA. Recent evidence from phase III clinical trials led to the approval of bevacizumab, an anti-VEGF monoclonal antibody, by the FDA as first line therapy in metastatic colorectal carcinoma in combination with other conventional chemotherapeutic agents known in the art.

Hence a composition according to present invention may comprise VEGF-inhibitor such as e.g. neutralizing monoclonal antibodies against VEGF or its receptor, small molecule tyrosine kinase inhibitors of VEGF receptors, soluble VEGF receptors which act as decoy receptors for VEGF and ribozymes which specifically target VEGF mRNA or any combinations thereof.

Other potentially useful VEGF or related inhibitors are monoclonal or polyclonal or recombinant antibodies classified as belonging to one or more of;
anti-VEGF A, B, C, D, E or F including all subtypes
anti-VEGF Receptor 1 or 2anti-FLT 1 or 4
anti-KDR
anti-NRP1 or 2
anti-VEGFR 1-4
anti-ARHGEF4
anti-Prokineticin1
anti-Neuropilin 1
anti-Corticotropin-Releasing Factor Receptor 2
anti-FIGF.

It is envisaged that any inhibitor of vessel formation can be used in combination with immunotherapy. Accordingly, the inhibitor may be an inhibitor of VEGF including an anti-VEGF antibody (e.g. monoclonal or polyclonal antibodies or recombinant antibodies) or fragments thereof. The inhibitors of vessel formation for use in combination with immunotherapy, notably the immunotherapy exemplified herein, include those specifically disclosed herein.

All details described in this paragraph under the heading "Vessel formation inhibitors, notably inhibitors of VEGF" are also valid for inhibitors of VEGF contained in a composition containing an immunotherapy component and an angiogenesis component.

### Combination therapy

The inventors have now surprisingly found that a combination, either as a single composition of one or several VEGF-inhibitors and CD4+ helper T-lymphocytes and/or CD8+ T-lymphocytes (e.g. obtainable by the method described herein), or as two separate preparations, one preparation comprising one or more VEGF-inhibitors and one preparation comprising the T-lymphocytes, administered to a subject in need thereof, can lead to one or more of the effects mentioned above.

The T-lymphocytes may be
CD4+ helper T-lymphocytes,
CD8+ T-lymphocytes,
Th1 cells.
memory cells derived from Th1 cells and/or
effector cells derived from Th1 cells,
and any combination thereof.

In practice it is almost impossible to obtain a 100% pure cell population only containing one specific type of T-lymphocytes. However, as seen from the examples herein, normally CD4+ and CD8+ T-lymphocytes constitute at least 35% of the cell population in the composition employed; often they constitute at least 60% and in most cases more than 85% of the cell population (as detected with flow cytometry). In the present context, the immunotherapy component of the composition is said to be a CD4+ helper T-lymphocytes component if it contains 51% or more CD4+ helper T-lymphocytes such as, e.g., 60% or more, 75% or more, 90% or more, or 95% or more. Analogous, the immunotherapy component of the composition is said to be a CD8+ T-lymphocytes component if it contains 51% or more CD8+ T-lymphocytes such as, e.g., 60% or more, 75% or more, 90% or more, or 95% or more.

For an effective treatment of cancer, administration of tumour-reactive T-lymphocytes of the Th1 type is especially beneficial, as this type is believed to promote activation of cytotoxic T lymphocytes (Tc), NK cells, macrophages, and monocytes, all of which can attack cancer cells and generally defend against tumours. I.e. in a specific embodiment the invention relates to a composition, wherein the immunotherapy component comprises at least 85% of tumour-reactive CD4+ helper T-lymphocytes. In general, the percentage of T-lymphocytes of the Th2 type generated by the method described in the reference example is 30% or less, such as, e.g., 25% or less, 20% or less, 15% or less, 10% or less, 5% or less or 0%, whereas at least 70% of the tumour-reactive CD4+ T-lymphocytes are of the Th1 type, such as, e.g. at least 75%, at least 80%, at least 85%, at least 90%, at least 95% or 100%.

As mentioned above, it is advantageous if the immunotherapy component contains a substantial amount of memory tumour-reactive CD4+ T-lymphocytes. The memory tumour-reactive CD4+ T-lymphocytes enables an up to life long protection of the patient towards recurrence of the tumour or metastasis of the primary tumour.

Accordingly, the present invention also provides a composition, wherein the immunotherapy component comprises memory T-lymphocytes. Normally, when a culture of tumour-reactive T-lymphocytes are expanded according to Reference Example 1 herein from about 35% to about 90% of tumour-reactive T-lymphocytes are of the memory type, such as, e.g. from about 40% to about 90%, from about 50% to about 80% or from about 60% to about 70%, will be obtained. The present inventors speculates that the fact that the lymphocytes in phase i) are allowed to be regenerated before tumour antigen is added, together with the relatively slow expansion phase leads to formation of a high ratio of memory lymphocytes to effector lymphocytes.

Notably, the present invention relates to a composition comprising relevant T-lymphocytes obtained by the two-step method disclosed in the Reference Example 1 hereinand one or more of: neutralizing monoclonal antibodies against VEGF or its receptor, small molecule tyrosine kinase inhibitors of VEGF receptors, soluble VEGF receptors which act as decoy receptors for VEGF and ribozymes which specifically target VEGF mRNA.

As mentioned herein before, there may be situations where the composition may be a single composition, i.e. a composition containing both the active principles, i.e. the tumour-reactive T-lymphocytes and the VEGF inhibitor. In such cases, the two active principles are present in a composition that is suitable for parenteral or local administration, notably in a medium that is harmless to the T-lymphocytes (i.e. the characteristics of the cells are maintained and the cells are viable) and in which the VEGF inhibitor is soluble or dispersible. Such a single composition can be adapted to be administered once or several times dependent with frequent or less frequent intervals on the specific type of cancer and the response to the treatment. Normally, it is envisaged that the composition is intended for administration once and, accordingly, it must contain an efficient amount of the two active principles (for dosages, see in the following). However, such a single composition requires that the disease to be treated benefits from simultaneous administration of the individual active principles (T-lymphocytes and VEGF inhibitor, respectively).

However, as described herein, administration of the active principles is normally separated in time and, accordingly, a composition according to the invention normally Is in the form of two separate preparations intended to be administered at the same time (or almost at the same time) or sequentially, i.e. one component is administered e.g. at time, 0, the other at a later time, t. Such a composition enables a greater variation with respect to dosage and administration forms. Thus, the immunotherapy component may be in the form of a parenteral preparation for injection or infusion, whereas the angiogenetic component may be in the form of an implant to be implanted e.g. subcutaneously or in a body cavity (e.g. vagina, bladder etc). Moreover, one of the components may be included in a form that makes it possible to administer a dose more than once daily. Thus, it is envisaged that a variety of combinations can be made without departing from the purpose with the invention.

At present, cell immunotherapy and antiangiogenic therapy both involve parenteral administration. However, due to risk of cell death it is currently believed that the composition should be stored in two separate preparations and either administered as such or relatively short time (up to 1 hour) before administration the two preparation should be mixed and administered. However, other administration routes are not excluded, cf. the following.

Administration of the composition according to the invention can be accomplished by various art-known routes such as enteral, parenteral, other parenteral or topical administration.

Examples of parenteral administration are, but not limited to:
intravenous (into a vein), intraarterial (into an artery), intramuscular, subcutaneous, intraosseous infusion, intradermal, intrathecal or intraperitoneal.

Parenteral administration can be achieved by injecting or infusing a drug composition intravenously, intra-arterially, intramuscularly, intrathecally, intradermally, intraperitoneally, subcutaneously etc.

Examples of other parenteral administrations are, but not limited to:
transdermal, transmucosal, buccal (absorbed through cheek near gumline), implants. Other means of administration may be, but are not limited to: epidural (peridural), or intravitreal.

Transdermal administration can be accomplished by applying, pasting, rolling, attaching, pouring, pressing, rubbing etc of a transdermal preparation to the skin or mucosa etc.

Examples of topical administration are, but not limited to:
epicutaneous (application onto the skin), inhalational, enema, eye drops (onto the conjunctiva), ear drops or vaginal.

Examples of enteral administration are, but not limited to:
oral, by gastric feeding tube, duodenal feeding tube, gastrostomy, or rectal.
An oral administration can be achieved by swallowing, chewing, sucking of an oral dosage form comprising the drug and its formulation.
An oral formulation may be conventional tablets, capsules, caplets, solutions, suspensions, emulsions etc.
Conventional tablets may be immediate or sustained release oral tablets. Tablets may also be designed so as to release the medical composition at specific tissues by applying for example different coating techniques. Tablets may thus be coated with films or sugars so as to control the rate and the site of release of the drug.

Currently, it is not envisaged that cell immunotherapy could be given e.g. by the oral route, but it cannot be excluded that e.g. cancer in the mouth or oesophagus can be treated via oral administration. Local administration to treat e.g. rectal cancer, colon cancer, bladder cancer, skin cancer etc. is also an option.

As mentioned above, the presently used administration route is the parenteral. Accordingly, this aspect is important.

It is understood that the administration comprising the composition according to the invention may be administered separately by any of the above mentioned administration pathways. When the composition comprises a kit with two separate preparations, one preparation comprising the relevant T-lymphocytes and one comprising one or more VEGF-inhibitors, administration may be performed such that the preparation comprising relevant T-cells may be administered parenterally and the preparation comprising one or more VEGF-inhibitors may be administered either parenterally or by any other relevant route depending on the method of administration best suited for the purpose.

The invention relates to a composition comprising relevant T-lymphocytes and one or more monoclonal antibodies against VEGF, wherein one monoclonal antibody is bevacizumab (Avastin^{®}). Other monoclonal antibodies are Ab-153, Ab-309, Ab-342 (see Chen J.H. et al, Biochemisry and molecular biology international 47(2): 161-9, 1999 Feb) or ranibizumab (Lucentis^{®}).

The invention also relates to a composition comprising relevant T-lymphocytes and one or more small molecule tyrosine kinase inhibitors, wherein the tyrosine kinase inhibitor may be, but is not limited to, sunitinib (Sutent), sorafenib (Nexavar), axitinib, and pazopanib, ABT-869, BIBF1120, 4-[(4-fluoro-2-methyl-1 H-indol-5-yl)oxy]-6-methoxy-7-[3-(pyrrolidin-1-yl)propoxy]quinazoline (Cediranib), -Amino-5-fluoro-3-[6-(4-methyl-1-piperazinyl)-1H-benzimidazol-2-yl]-2(1H)-quinolinone (Dovitinib), 3-pyridinecarboxamide, N-(2,3-dihydro-3,3-dimethyl-1H-indol-6-yl)-2-[(4-pyridinylmethyl)amino]-N-(3,3-dimethyl-2,3-dihydro-1H-indol-6-yl)-2-[(pyridin-4-ylmethyl)amino]pyridine-3-carboxamide (Motesanib) or [N-(4-bromo-2-fluorophenyl)-6-methoxy-7-[(1-methylpiperidin-4-yl)methoxy]quinazolin-4-amine (Vandetanib) or cetuximab or any pharmaceutically acceptable salt thereof

A composition of the invention may comprise relevant T-lymphocytes and one or more ribozymes which specifically target VEGF mRNA, wherein the ribozymes may be, but is not limited to, anti-Fit-1 ribozymes. One such example is Angiozyme^{®} (US 6,346,398). This aspect also covers other hammerhead ribozymes.

A composition of the invention may comprise additional pharmaceutically active compounds used in conventional chemotherapy. The composition may thus comprise, but is not limited to, compounds used in Avastin therapy. The composition may thus also comprise, but is not limited to, 5-fluorouracil, leucovorin, oxaliplatin, paclitaxel, docetaxel, fumagallin, anthracyclines such as daunorubicin, doxorubicin and epirubicin, camptothecins such as irinotecan and topotecan, vincristine, carboplatin, cisplatin, cyclophosphamide, mitomycin C, mitoxanthrone, floxuridine, gemcitabine, methotrexate, bleomycin, etoposide, vinblastine, vindesine, vinorelbine and genistein.

The present invention also relates to use of a composition comprising relevant T-lymphocytes and one or more of: neutralizing monoclonal/polyclonal/recombinant antibodies against VEGF or its receptor, small molecule tyrosine kinase inhibitors of VEGF receptors, soluble VEGF receptors which act as decoy receptors for VEGF and ribozymes which specifically target VEGF mRNA, and optionally one or more chemotherapeutic agents selected from, but not limited to 5-fluorouracil, leucovorin, oxaliplatin, paclitaxel, docetaxel, fumagallin, anthracyclines such as daunorubicin, doxorubicin and epirubicin, camptothecins such as irinotecan and topotecan, vincristine, carboplatin, cisplatin, cyclophosphamide, mitomycin C, mitoxanthrone, floxuridine, gemcitabine, methotrexate, bleomycin, etoposide, vinblastine, vindesine, vinorelbine and genistein in the manufacture of a medicament in the treatment of cancer.

The invention also relates to use of a composition comprising relevant T-lymphocytes and one or more monoclonal antibodies against VEGF, wherein one monoclonal antibody is bevacizumab (Avastin^{®}) in the manufacture of a medicament in the treatment of cancer. In addition to bevacizumab Ab-153, Ab-309, Ab-342 or ranibizumab (Lucentis^{®}) may be used.

The compositions of the invention may comprise one or more additional pharmaceutical excipients. Especially, the immunotherapy component may comprise a medium that is suitable for maintaining the characteristics and viability of the cells, while it at the same time is suitable for injection into a mammalian (human) body (i.e. it must be safe). A suitable medium may be isotonic saline (0.9% w/w sodium chloride) optionally supplemented with serum albumin, notably human serum albumin, and/or pH adjusting agents such as, e.g., a phosphate buffer.

Dependent on the specific preparation or composition, the excipient can be selected e.g. from, but not limited to,
- for parenteral administration: solvents including water and propylene glycol, vegetable oils, osmotic pressure adjusting agents, pH adjusting agents, serum-free media, cell nutritional solutions or agents, human or artificial serum albumin, autologous serum, isotonic media which may optionally have the same tonicity as blood, physiological saline solutions etc.
- for enteral administration: antiadherents, binders, coatings, disintegrants, fillers/diluents, flavours and colours, pH-regulating agents, glidants, lubricants, preservatives, sorbents, sweeteners and agents that change the dissolution rates of active species, serum free media, buffering agents or solutions, cell nutritional solutions or agents, pH-regulating agents, human or artificial serum albumin, autologous serum, isotonic media which may optionally have the same tonicity as blood, physiological saline solutions, preservative agents.

The composition according to the present invention may be used in the treatment of any solid neoplasm of epithelial, mesenchymal or embryological origin in any anatomical location, such as e.g., for epethilal neoplasms e.g. carcinomas in the breast, colon, pancreas, bladder, small intestines, prostate, cervix, vulva, ovaries; for mesenchymal neoplasms e.g. sarcomas in the joints, bones, muscles and tendons and some haematological such as lymphomas; for embryological neoplasms, e.g. teratomas. More specific examples are, but not limited to, colorectal cancer, metastatic colorectal cancer, breast cancer, metastatic breast cancer, metastatic renal cell carcinoma, metastatic glioblastoma multiforme, metastatic ovarian cancer, metastatic hormone-refractory prostate cancer, unresectable locally advanced pancreatic cancer renal cell cancer, ovarian cancer, refractory non-small cell lung cancer and lung cancer.

Notably, the treatment described herein is suitable for treatment of cancer selected from carcinomas in the breast, colon, rectum, pancreas, liver, bile bladder, bile ducts, urinary bladder, small intestines, lung, prostate, kidney, cervix, vulva, ovaries, malignant melanoma, head and neck carcinomas; sarcomas in the joints, bones, muscles and tendons, lymphomas; teratomas; and metastases and locally advanced (inoperable) of the above-mentioned tumours.

The composition of the invention may comprise cancer type specific CD4+ T lymphocytes, wherein at least 70% of cancer specific CD4+ T lymphocytes are of the Th1 type and 30% or less of cancer specific CD4+ T lymphocytes are of the Th2 type and one or more VEGF-inhibitors and optionally an additional pharmaceutically active compound used in Avastin^{®} therapy.

The composition of the invention may also comprise cancer specific T lymphocytes, wherein from about 35% to about 90% are of the memory type and one or more VEGF-inhibitors and optionally an additional pharmaceutically active compound used in Avastin^{®} therapy.

The composition of the invention may also comprise cancer specific T lymphocytes, wherein from about 10% to about 65% are effector T-lymphocytes and one or more VEGF-inhibitors and optionally an additional pharmaceutically active compound used in Avastin^{®} therapy.

The composition of the invention may comprise cancer specific T lymphocytes wherein the composition comprises at least 1 million, at least 5 millions, at least 10 million or at least 20 million or at least 30 million or at least 40 million or at least 50 million or at least 100 million cancer specific T-lymphocytes and one or more VEGF-inhibitors and optionally an additional pharmaceutically active compound used in Avastin^{®} therapy. The dosage of cells depends on the severity of the disease. Moreover, due to shortage of tumour-reactive T-lymphocytes, a lower dose may also be used.

The composition according to the invention may comprise a VEGF-inhibitor in the amount of from about 1mg to about 2000 mg or from about 70 mg to about 1400 mg or from about 140 mg to about 1050 mg or from about 210 mg to about 1050 mg, or from about 280 mg to about 1050 mg or from about 350 mg to about 1050 mg.

The composition according to the invention may comprise a VEGF-inhibitor is the dose of; from about 0,1 to about 50 mg/kg body weight, or from about 0,5 to about 30 mg/kg body weight or from about 1 mg/kg to about 20 mg/kg body weight or from about 2 mg/kg to about 15mg/kg body weight, or from about 3 mg/kg to about 15 mg/kg body weight, or from about 4 mg/kg to about 15 mg/kg body weight, or from about 5 mg/kg to about 15 mg/kg body weight.

The above data is the doses normally used for Avastin^{®} and are envisaged to be of the same order of magnitude for other VEGF-inhibitors. However, a person skilled in the art will know how to determine the correct dose. Moreover, it is envisaged that the dosage of the VEGF-inhibitor can be slightly reduced e.g. with about 10% or more such as e.g. about 20% or more, such as e.g. about 30% or more, such as e.g. about 40% or more, such as e.g. about 50% or more, such as about 60% or more, such as e.g. about 70% or more, such as e.g. about 80% or more. For example, in a reduction of about 10%, doses from 50 mg to 1250 mg are expected to be sufficient.

It is understood that the composition according to the present invention, may be a composition comprising a therapeutic effective amount of tumour reactive T-lymphocytes and a therapeutic effective amount of one or more VEGF-inhibitors, one VEGF-inhibitor being bevacizumab (Avastin®).

It is also understood that a composition according to the present invention, may be a composition comprising two separate preparations wherein one preparation comprises a therapeutic effective amount of tumour reactive T-lymphocytes and the second preparation comprises a therapeutic effective amount of one or more VEGF-inhibitors. A therapeutic effective amount of one or more chemotherapeutic agents, if present, may be included in a further separate preparation or it may be included in the preparation comprising one or more VEGF inhibitors, one VEGF-inhibitor being bevacizumab (Avastin®).

It is understood that the composition according to the present invention may be a kit comprising two separate preparations wherein one preparation comprises a therapeutic effective amount of tumour reactive T-lymphocytes and the second preparation comprises a therapeutic effective amount of one or more VEGF-inhibitors. The kit may optionally comprise a chemotherapeutic agent as a separate composition in the kit or combined in any order with either the relevant T-lymphocytes or one or more VEGF-inhibitors.

### Method of treatment

A combination of one or more VEGF-inhibitors and tumour-reactive T-lymphocytes as described herein before may be used in treating diseases of neoplastic origin. All details and particulars described herein regarding VEGF-inhibitors, T-lymphocytes, compositions, administration routes, dosages etc apply mutatis mutandis to the method aspect of the present invention.

The tumour-reactive T-lymphocytes obtained by the expansion method as described herein and one or more VEGF-inhibitors may be used in a method for treating a subject suffering from a disease of neoplastic origin or for effecting tumour regression in a subject having a tumour, the method comprising administering to the subject in need thereof an effective amount of tumour-reactive T-lymphocytes according to the invention and an effective amount of one or more VEGF-inhibitors, one VEGF-inhibitor being bevacizumab (Avastin®).The method may comprise administration of an effective amount of tumour-reactive T-lymphocytes according to the invention and an effective amount of one or more VEGF-inhibitors as a single composition or may be administered as separated preparations, wherein administration to a subject in need thereof may be simultaneous or sequential in any order. The method also comprises administration of the tumour-reactive T-lymphocytes as a first therapeutic method and thereafter a second therapeutic method comprising administration of the tumour-reactive T-lymphocytes in combination with one or more VEGF-inhibitors, one VEGF-inhibitor being bevacizumab (Avastin®).

The method described herein may be used for treatment of any solid neoplasm of epithelial, mesenchymal or embryological origin in any anatomical location, such as e.g., for epethilal neoplasms e.g. carcinomas in the breast, colon, pancreas, bladder, brain, small intestines, prostate, cervix, vulva, ovaries; for mesenchymal neoplasms e.g. sarcomas in the joints, bones, muscles and tendons and some haematological such as lymphomas; for embryological neoplasms, e.g. teratomas. More specific examples are, but not limited to, colorectal cancer, metastatic colorectal cancer, breast cancer, metastatic breast cancer, metastatic renal cell carcinoma, metastatic glioblastoma multiforme, metastatic ovarian cancer, metastatic hormone-refractory prostate cancer, unresectable locally advanced pancreatic cancer, renal cell cancer, ovarian cancer, refractory non-small cell lung cancer and lung cancer.
The definition of an effective amount of tumour-reactive T-lymphocytes is depending on the specific type of lymphocytes, the ratio of memory to effector T-lymphocytes and on the severity of the disease. However, in average a minimum of at least 1 milllion, at least 5 millions, at least 10 million, such as, e.g. at least 20 million, at least 30 million, at least 40 million, at least 50 million, at least 60 million, at least 70 million, at least 80 million or at least 100 million tumour-reactive T-lymphocytes may be administered. The present inventors have not identified any upper limit with respect to the amount of tumour-reactive T-lymphocytes to be administered in a single dose.

The tumour-reactive T-lymphocytes for administration may comprise a combination of effector T-lymphocytes and memory T-lymphocytes. More specific the amount of tumour-reactive T-lymphocytes of the memory type may be from about 35% to about 90%, such as, e.g. from about 40% to about 90%, from about 50% to about 80% or from about 60% to about 70%, and a percentage of effector T-lymphocytes from about 10% to about 65%, such as, e.g., from about 20% to about 50% or from about 30% to about 40%.

The definition of an effective amount of VEGF-inhibitor is depending on the severity and type of the disease. However, the normal dose administered is in the range of 1 mg/kg to about 20 mg/kg, such as 2mg/kg to about 15mg/kg, such as 3 mg/kg to about 15 mg/kg, such as 4 mg/kg to about 15 mg/kg, such as 5 mg/kg to about 15 mg/kg.

The tumour-reactive T-lymphocytes for administration may comprise a combination of effector T-lymphocytes and memory T-lymphocytes. More specific the amount of tumour-reactive T-lymphocytes of the memory type may be from about 35% to about 90%, such as, e.g. from about 40% to about 90%, from about 50% to about 80% or from about 60% to about 70%, and a percentage of effector T-lymphocytes from about 10% to about 65%, such as, e.g., from about 20% to about 50% or from about 30% to about 40% and one or more VEGF-inhibitors in the dose of 1 mg/kg to about 20 mg/kg, such as 2mg/kg to about 15mg/kg, such as 3 mg/kg to about 15 mg/kg, such as 4 mg/kg to about 15 mg/kg, such as 5 mg/kg to about 15 mg/kg body weight (notably with a reduction of at least 10%, cf. above).

The tumour-reactive T-lymphocytes and the VEGF-inhibitors may be formulated as a pharmaceutical composition suitable for parenteral administration to the patient such as, e.g., intravenous, intraarterial, intrathecal, or intraperitonal administration or orally by administrating of a formulation in conventional tablet form, capsules, caplets, solutions, suspensions or emulsions.

When the tumour-reactive T-lymphocytes are administered parenterally, they may be formulated in an isotonic medium, i.e. in a medium having the same tonicity as blood, and comprising one or more substances preventing aggregation of the cells and may comprise additional pharmaceutical excipients. A specific example of a suitable medium is a 0.9% NaCl solution comprising up to 3% human serum albumin such as, e.g. up to 2% human serum albumin or up to 1% human serum albumin. For intravenously administration the concentration of tumour-reactive T-lymphocytes in the composition to be administered normally lies within the range from about 0.5 million lymphocytes/ml medium to about 4 million lymphocytes/ml medium, such as, e.g., from about 0.5 million lymphocytes/ml medium to about 3 million lymphocytes/ml medium, from about 0.5 million lymphocytes/ml medium to about 2 million lymphocytes/ml medium or from about 1 million lymphocytes/ml medium to about 2 million lymphocytes/ml medium.

When the VEGF-inhibitors are administered parenterally, they may be formulated in an isotonic medium, i.e. in a medium having the same tonicity as blood, and may comprise additional pharmaceutical excipients. A specific example of a suitable medium is a 0.9% NaCl solution comprising up to 3% human serum albumin such as, e.g. up to 2% human serum albumin or up to 1% human serum albumin. For intravenously administration the dose of the VEGF-inhibitors in the composition to be administered normally lies within the range from about 1 mg/kg to about 20 mg/kg, such as 2mg/kg to about 15mg/kg, such as 3 mg/kg to about 15 mg/kg, such as 4 mg/kg to about 15 mg/kg, such as 5 mg/kg to about 15 mg/kg (notably with a reduction of at least 10%, cf. above). For the oral or other administration routes the dose is contemplated to be of the same order of magnitude or up to 50% larger.

The composition comprising the VEGF-inhibitors may be administered as a single dose or multiple doses. It may be infused over 1 to 2 hours or more The composition comprising tumour-reactive T-lymphocytes may be administered as a single dose or multiple doses. It may be infused over 1 to 2 hours or more.

The composition according to the invention may be formulated as a single composition comprising the tumour reactive T-lymphocytes and one or more VEGF-inhibitors as a single composition or as a two separate preparations to be used in a combination (either simultaneously or sequentially in any order), one preparation comprising the tumour reactive T-lymphocytes and one preparation comprising one or more VEGF-inhibitors.

The treatment may be performed once or repeated depending on the severity of the disease. Furthermore, the treatment may be reiterated upon recurrence of the disease or with any interval which is considered necessary depending upon the severity and character of the disease to be treated.

The treatment according to the present invention may be supplemented with any other relevant treatment for cancer. Such supplemental treatment may be given before, at the same time or after the administration of the lymphocytes and one or more VEGF-inhibitors and it may be given at frequencies normally used for such treatments. A suitable example of supplemental treatment is chemotherapy and the like. Chemotherapeutic agents may be, but are not limited to, 5-fluorouracil, leucovorin, oxaliplatin, paclitaxel, docetaxel, fumagallin, anthracyclines such as daunorubicin, doxorubicin and epirubicin, camptothecins such as irinotecan and topotecan, vincristine, carboplatin, cisplatin, cyclophosphamide, mitomycin C, mitoxanthrone, floxuridine, gemcitabine, methotrexate, bleomycin, etoposide, vinblastine, vindesine, vinorelbine and genistein.

Administration of the above mentioned compositions may be in form of a kit, wherein the kit may be a single composition or a kit of two or three single compositions. The different single compositions may be administered simultaneously or sequentially in any order with any time interval. Administration of the kit components may be independently oral or parenteral in any order.

As demonstrated in the Examples herein the combination therapy may be displaced in time and still excellent results are achieved. Thus, the combination therapy may be initiated with
i) administration of tumour-reactive T-lymphocytes followed by
ii) administration of a VEGF inhibitor optionally in combination with a chemotherapeutic cocktail (one or more chemotherapeutic agents, e.g. in the treatment of colon cancer a combination of 5-fluoruracil, leucovorin and oxaliplatin or irinotecan; this regime normally follows the scheme traditionally applied for such combinations, i.e. with respect to administration frequency and dosing.

The two administration regimes may be displaced in time by at least 15 days, notably from 1-90 days such as from 15-60 days including 30 days or 1 month.

In the following is described two different preferred procedures, where two different modes of action can be achieved.

Preferred procedure 1: Chemotherapy is administered by infusion approx. four weeks before harvest of Sentinel nodes. Chemotherapy is administered according to adequate dose and procedure for the relevant drug. This chemotherapy step of the procedure is optional.

After approx. another four weeks after harvest the Sentinel or metinel node aquired lymphocytes (SNALs or MNALs) are administered. Optimal dosage of SNALs or MNALs is unknown, but effect has been seen from infusion of 50 million lymphocytes. Optimal dosage is probably higher as effect is believed to be correlated with number of infused SNALs or MNALs. Infusions of more than 1 billion lymphocytes has been tolerated by patients. Approximate time of the infusion procedure for lymphocytes is one (1) hour.

After approx. another three weeks, VEGF inhibitors are administered by infusion according to adequate doses for the relevant drug.

In this treatment regimen, the VEGF inhibitor can be believed to cause increased inflammation in vessels, thereby causing increased recruitment/homing of tumour reactive SNALs or MNALs.

Preferred procedure 2: VEGF inhibitor is administered approx. three weeks before harvest of Sentinel or Metinel nodes. As above, SNALs or MNALs are administered approx. four weeks after harvest. Dosage etc. as above for both drugs.

Chemotherapy is not administered in this regimen.

The mode of action for this regimen is anticipated increase in number of harvested SNALs or MNALs due to increased tumour cell death and thereby increased antigen presentation in Sentinel or Metinel node.

As mentioned above, a chemotherapeutic cocktail may be included as well (the nature depends on the specific cancer to be treated).

As a follow-up treatment both i) and ii) may be given or only one of i) and ii) may be administered. Excellent results were obtained when i) was given 30 days after initiation of ii).

Alternatively, a combination therapy may be initiated with
i) administration of a VEGF inhibitor optionally in combination with a chemotherapeutic cocktail (one or more chemotherapeutic agents, e.g. in the treatment of colon cancer a combination of 5-fluoruracil, leucovorin and oxaliplatin or irinotecan; this regime normally follows the scheme traditionally applied for such combinations, i.e. with respect to administration frequency and dosing followed by
ii) administration of tumour-reactive T-lymphocytes.

The two administration regimes may be displaced in time by at least 15 days, notably from 1-90 days such as from 15-60 days including 30 days or 1 month.

As a follow-up treatment both i) and ii) may be given or only one of i) and ii) may be administered.

### Description of the drawing

Figure 1 illustrates that the sentinel node is the natural primary site for the presentation and activation of T cell reactivity towards tumour antigen.
Figure 2 shows that initially sentinel node lymphocytes are activated with tumour antigen and low dose IL-2 resulting in activation and expression of the activation marker CD25 (Top panel). The end of phase I activation phase is defined by the decreased number of CD4⁺ T cells expressing CD25 (Bottom panel). When less than 5% of the CD4⁺ T cells express CD25 phase II is initiated with restimulation with antigen.
Figure 3 illustrates that Phase I and Phase II activation results in expansion and enrichment of CD4⁺ T helper cells.
Figure 4 illustrates that in Phase I the majority of cells are naïve CD62L+ cells or activated CD69+CD62L+ cells. After Phase II the majority of the cells are CD62L- and are composed of memory and effector CD4+ T helper cells. CD62L- T cells are not expressing the preferred lymph node homing molecule, thus they are seeking inflammatory areas in non-lymphatic organs.
Figure 5 shows primary cells stimulated in Phase I from the tumour (Tumour infiltrating lymphocytes), sentinel nodes (SN) and an irrelevant lymph node (LN) results in no little IFN-γ production.
Figure 6 illustrates that after expansion after phase ii) there is a dose dependent increase in antigen dependent IFN-γ production.
Figure 7 illustrates that the expansion and activation protocol promotes the expansion of antigen specific T cell clones as investigated by the selective enrichment of TCR Vβ expression.

### Examples

### Reference Example 1

### Expansion of tumour-reactive T-lymphocytes

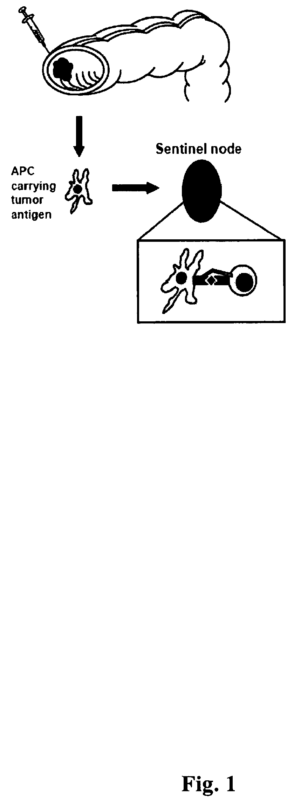

Identification of sentinel nodes was done peroperatively using the sentinel node technique. Briefly, 1 ml of Patent blue dye was injected (Guerbet, Paris) and distributed superficially in the serosa around the tumour. Within five to ten minutes, one to three mesenteric lymph nodes were coloured blue, these sentinel nodes were marked with sutures and removed (see Figure 1). One non-sentinel mesenteric lymph node, distant from the tumour, was also identified and removed as a control.

The sentinel- and non-sentinel lymph nodes were cut in half and 1 mm thick slices were taken from the centre and the periphery. The rest of the lymph nodes were sent for histopathological examination according to routine procedure. A part of the tumour, including a sample of the invasive margin, was also removed for research purposes.

### Cell culture

### Phase I, initial activation

The sentinel node material was kept on ice and immediately taken care of using AIM V® Media (Invitrogen) at all times. Single cell suspensions of sentinel node lymphocytes were obtained through gentle homogenisation in a loose fit glass homogenisator, and following homogenisation cells were washed twice in medium. The sentinel node lymphocytes were put in cell culture flasks at 4 million cells/ ml and interleukin-2 (IL-2) (Proleukin®, Chiron) was added to a concentration of 240 IU/ml medium.

Autologous tumour extract was prepared by homogenisation with an Ultra Turrax in 5 volumes (w/v) 2 x PBS followed by denaturation for 5 minutes at 97°C. Three to four days after initiation of the cell culture autologous tumour extract was added at a concentration of 1/100. For long-term culture the cells were kept in a cell incubator at 37°C and 5% CO₂ and 240 IU IL-2/mL media added every 3-4 days.

### Phase II, activation and expansion

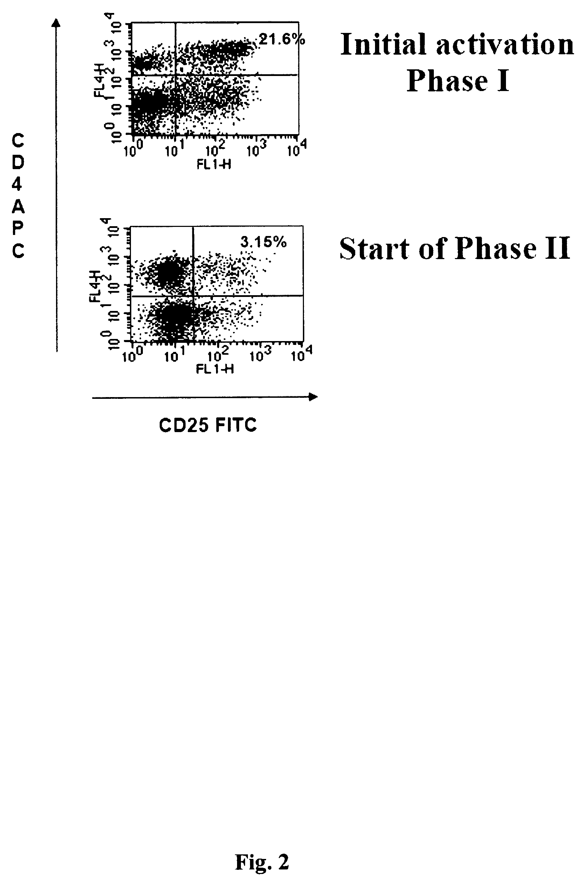

After 18-22 days the cell cultures were monitored for the expression of CD25. When the number of CD25 expressing cells was diminished below 5% the cells were restimulated in Phase II (Figure 2) by the addition of autologus tumour extract at a concentration of 1/100. For efficient antigen presentation autologous PBMC were collected using Ficoll-Paque PLUS (Amersham Biosciences, GE Healthcare), radiated with 2500 rad and added to the cell cultures. Three days after restimulation interferon-α (Introna) in conc. 100-500 IU/ml and anti IL-4 antibody was added to a concentration of 2 µg/ml. After 5 to 8 days IL-12 (4 ng/ml) was added to the expansion in order to promote induction of IFN-γ producing Th1 cells.

The day before transfusion to the patient the cell cultures were subject to purification using a Ficoll-Paque PLUS (Amersham Biosciences, GE Healthcare) in order to retrieve the viable cells in the culture. On the day of transfusion the cells were washed twice in Saline solution (Sodium chloride Baxter, Viaflo 9 mg/ml, Baxter) and then transferred to a transfer bag containing 100-200 ml of saline solution and 1% Human Serum Albumin (Baxter). Investigations for microbial presence were performed prior to transfusion. Infusions of the cells were performed during 1-2 hours under professional medical supervision.

### Immunological evaluation

Further immunological evaluation was performed using tritium labelled thymidine incorporation proliferation assays. An aliquot of Sentinel node lymphocytes was set aside for this purpose, a single cell suspension of non-sentinel node lymphocytes was obtained by gentle pressure in a loose fit glass homogenisator and peripheral blood leukocytes were purified by Ficoll-Paque PLUS (Amersham Biosciences, GE Healthcare).

Cells were resuspended and washed twice in RPMI 1640 (Life technologies) containing 2.5% fetal calf serum (FCS) (Life technologies). Finally, cells were resuspended in RPMI 1640 proliferation media containing 10% human AB serum (Sigma), 1% penicillin-streptomycin (Sigma) and 1% glutamine (Sigma). Lymph node cells and purified PBL were used at 3x10⁵ cells/well in a 96-well plate and stimulated with tumour homogenate diluted 1/100, 1/10 or Con A 10 µg/ml (Sigma) in triplicates. Proliferation was measured on day 5, 6 and 7 by adding 1 µCi of ³H-Thymidine/well (Amersham) 18 hours prior to harvesting. Samples were subjected to scintillation counting.

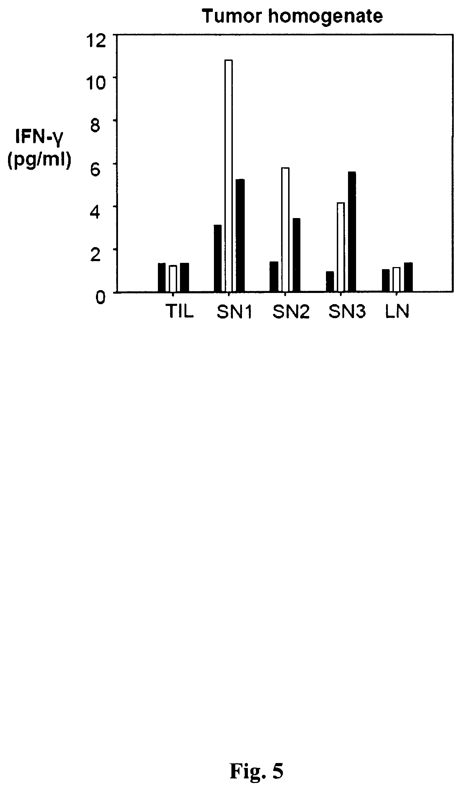

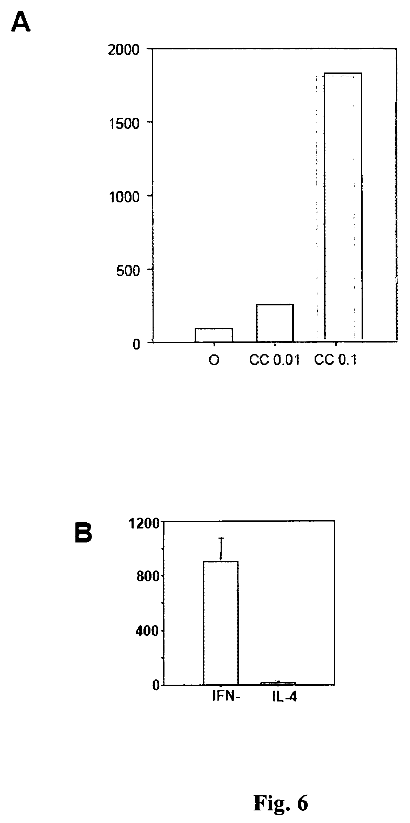

At the start of cell culture, stimulations of lymph node cells and PBL, for the measurement of IFN- y secretion, were performed in 96-well plates with 3x10⁵ cells/well in triplicate with tumour homogenate diluted 1/10 and 1/100, or Con A 10 µg/ml (Sigma). The amount of secreted IFN- y was measured with ELISA (Human IFN-γ Duoset, R&D Systems) on culture supernatants in pooled samples of the triplicates (Figure 5). At the end of cell cultures samples of the supernatant was removed and IFN- y and IL-4 secretion measured in triplicates with ELISA (Human IFN- Duoset and Human IL-4 Duoset, R&D Systems) (Figure 6 A and 6B).

### Flow cytometry analyses

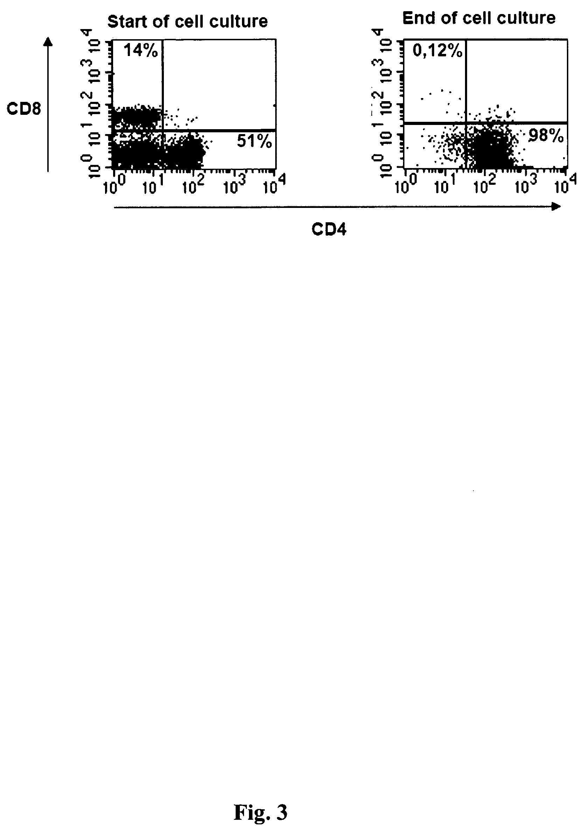

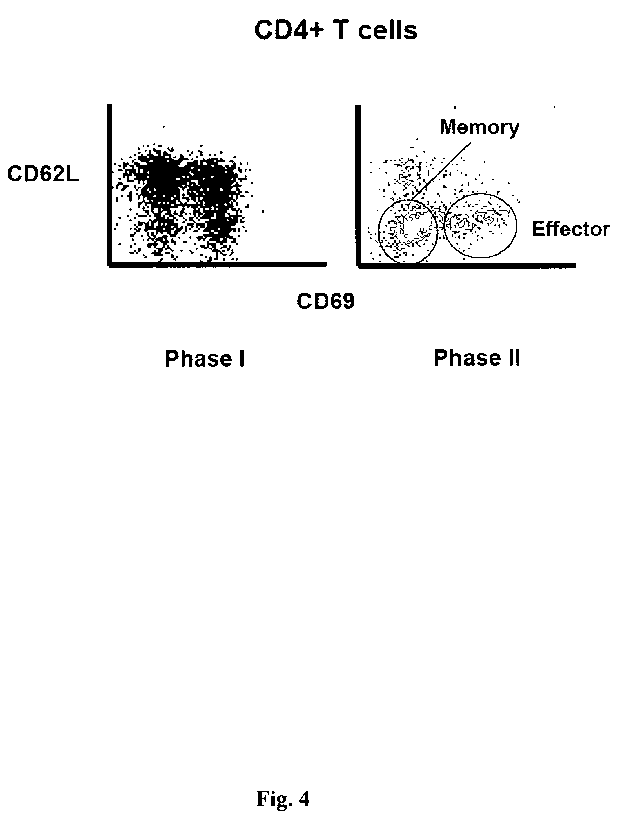

Characterisation of cells was performed using flow cytometry initially on cells from the sentinel node, non-sentinel node, PBMC and from the tumour. From the sentinel node acquired lymphocytes in culture samples were taken every two to three weeks for flow cytometry analyses. Cells were incubated for 30 minutes in PBS supplemented with 2% FCS and 0.05% NaN₃ (FACS buffer) with antibodies against markers for immune cell subpopulations and for lymphocyte activation (Figure 3, 4 and 5). Antibodies conjugated with Fluorescein isothiocyanate (FITC) against the following markers were used: CD69, HLA-DR, CD45RA, CD25, conjugated with phycoerythrin (PE): CD62L, CD19, CD45RO, CD56, conjugated with Peridinin-Chlorophyll-Protein (PerCP): CD8, CD3, conjugated with allophycocyanin (APC): CD4, CD14, CD8.

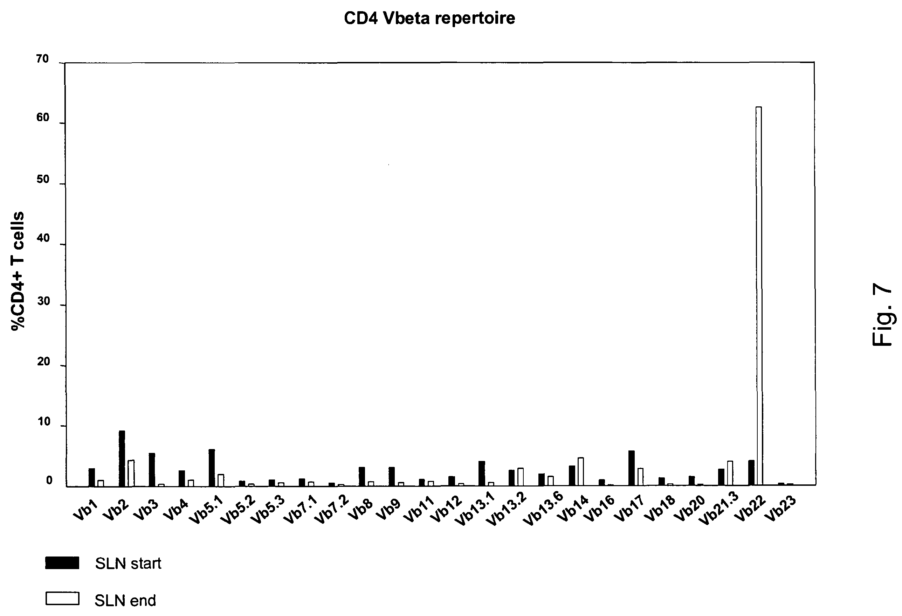

The Vβi-repertoire was examined using the Beta mark kit (Beckman Coulter), 5x10⁵ cells/tube was stained in 10 µl of the 8 different vials containing mixtures of FITC, PE and dual-colour FITC-PE conjugated TCR Vβ antibodies and with the addition of CD8 PerCP and CD4 APC to each tube (Figure 7).

### Reference Example 2

**Treatment of colon cancer by administering tumour-reactive T-lymphocytes**

### Identification and removal of sentinel and metinel lymph nodes from colon cancer patients

Sixteen patients diagnosed with colon cancer, six woman and ten men with an average age of 62 years were studied. Patients were histopathologically classified as Duke's C or D. There were also 5 patients with Duke's B with aggressive tumour characteristics such as ulcerations, vascular or perineural invasion. Patients 7 and 14 however had earlier been surgically treated due to colon cancer and now had recurrent disease with metastases to the liver. The local ethical committee approved the study and each patient gave informed consent.

Identification of sentinel or metinel nodes was done intraoperatively. Mobilisation of the colonic tumour site was achieved by division of peritoneal adhesions in order to facilitate inspection of tumour and mesentery. Injections of Patent blue dye (Guerbet, Paris) were distributed superficially in the serosa around the tumour. Within five minutes, one to three mesenteric lymph nodes were coloured blue, these sentinel nodes were marked with sutures and removed when the resection was complete. One non-sentinel mesenteric lymph node, distant from the tumour, was handled the same way.

The sentinel- and non-sentinel lymph nodes were cut in half and 1 mm thick slices were taken from the centre and the periphery. The rest of the lymph nodes were sent for histopathological examination according to routine procedure. A piece of the tumour, including a part of the invasive margin, was used for antigen preparation.

The lymphocytes obtained from the lymph nodes were then expanded as described in Reference Example 1.

### Administration of tumour-reactive T-lymphocytes

16 patients were treated with infusion of autologous lymphocytes expanded as described in Example 1. On average 74.7 million activated and clonally expanded T cells were administered as a transfusion. No toxic side effects like fever, chills, malaise, severe fluid retention, pulmonary oedema or respiratory distress were observed.

### Follow-up evaluations

Follow-up included clinical examination every third to sixth month and control of CEA levels. All stage III and IV patients were in addition investigated with computer tomography of the thorax and abdomen. The patients were followed for 36 months on average (range 6-51), and monitored in accordance with the Swedish colorectal cancer follow-up protocol. Out of the 16 patients who had been treated with infusion of autologous lymphocytes eight had known distant metastases at diagnosis. Four patients received their transfusions due to known recurrences and out of them three are still without signs of recurrences. One patient was operated due to a solitary liver metastases and has since then been without relapse. One patient with liver metastases located in both lobes (which had been declared incurable by liver surgery) had total regress of liver metastases after transfusion of tumour-reactive lymphocytes, and furthermore had normalisation of CEA levels, disappearance of ascites and is physically well fit, and exercising regularly. One further patient with liver metastases had regress of liver metastases and ascetic fluid after transfusion. One patient had three months after transfusion regress of metastases in the liver and lungs with almost a normalised CEA level at 5.9 (Normal < 4.0), disappearance of ascites and he appears clinically healthy.

### Results

Sixteen patients with colon cancer or solitary colorectal liver metastases were operated on at the South Stockholm General Hospital and included in the study. The primary locations of the tumours were three in caecum, 4 in colon ascendens, 1 in colon descendens, 7 in the sigmoid colon and 1 in rectum. Seven right-sided hemicolectomies, 1 left-sided hemicolectomy, 7 sigmoid resections and 1 rectumamputation were performed. Two patients had earlier been operated on with rectumamputation and sigmoid resection; they now underwent partial liver resections due to liver metastases. One patient had recurrences at two abdominal locations and had earlier been operated due to a tumour in the caecum. At our operation two sentinel nodes draining the metastasis were identified, one in the colonic mesentery and one in the mesentery of the small intestine. An extended resection of the anastomotic ileocolonic region with mesentery was done.

In all patients, one to three (average 2.1) sentinel node(s) were identified intraoperatively by peritumoural patent blue injections. Among the patients with primary colonic resection on average 15.8 lymph nodes were retrieved from each specimen. After histopathological investigation of these lymph nodes five patients were classified as Duke's C and 5 patients as Duke's B, all of them were classified as high-risk tumours due to growth of tumour cells along nerves and in vessels at pathological anatomical investigation. Five patients had distant metastases and were at time of metastatic resection classified as Duke's D. Two patients of them had solitary liver metastases. In addition sentinel nodes were also analysed by FACS (Fluorescence activated cell sorter) and antibodies against cytokeratin 20, which is expressed by colon cancer tumours, for the purpose to detect micrometastases. The cytokeratin 20 assessments of lymph nodes by flow cytometry were in agreement with the pathological anatomical diagnosis (not shown) except in one case where a false negative sentinel node (according to histopathological analysis) was positive in the cytokeratin 20 FACS analysis.

The sentinel node is the first lymph node draining the tumour and is therefore the first site of lymph node metastasis (Dahl et al, Eur. J. Surgical Oncology, 2005, 31, 381-385), but the sentinel node is also the primary site for the activation of the immune system. Tumour cells, debris, necrotic cells and antigen presenting cells accumulate in the sentinel node where presentation, activation and clonal expansion of T cells directed against the tumour occur. The present inventors took advantage of this population of *in vivo* expanded T cell population of sentinel node acquired lymphocytes for *in vitro* cell culture, expansion and transfusion.

Sentinel node acquired lymphocytes is a population of T cells activated and clonally expanded against tumour antigens that can efficiently be harvested during the surgical procedure. In contrast to recent immunotherapy trials focusing on cytotoxic T cells, the aim of the present inventors was to create a protocol for *in vitro* enhancement of the *in vivo* initiated clonal expansion of T helper cells. T helper cells seem to be necessary for the effective function of cytotoxic T cells and for the creation of memory cells. Furthermore, in a T cell receptor transgenic system targeting an islet cell antigen, the transfusion of Th1 cells was found to be sufficient for the β cell destruction and development of diabetes mellitus. *In vitro* culture of sentinel node acquired lymphocytes resulted in a Th1 activation and clonal expansion of T helper cells as indicated by the dominant production of the hallmark Th1 cytokine IFN-γ and the enrichment of a restricted TCR Vβ repertoire. The tumour homogenate used to expand the T cells is likely to be endocytosed and processed by antigen presenting cells for class II presentation leading to activation of CD4⁺ T helper cells resulting in expansion favouring T helper cells. By cross presentation antigens taken up by endocytosis may be processed and presented in the class I pocket resulting in activation of CD8⁺ cytotoxic T cells. Interestingly, in some cases the inventors found clonal expansion of both CD4⁺ and CD8⁺ T cells.

The average number sentinel node acquired lymphocytes at start of expansion was 107.4 million cells (range 3.6-509 millions, median 70 millions). Cells were characterised by flow cytometry. The ratio between CD4⁺ and CD8⁺ cells at start was in average 4.9 (range 0.36-10, median 5.4) indicating an expansion CD4⁺ T helper cells in sentinel nodes compared to the CD4/CD8 ratio in peripheral blood (normal range 1.0-2.5) (figure 2A). In addition B lymphocytes (CD 19) and natural killer (NK) cells (CD 56) were present in sentinel nodes (not shown). The cells were held in culture in average 36.1 days (range 23-58 days), median 33 days. Cells were monitored closely by flow cytometry at least weekly. Initially the total number of cells decreased. B cells and NK cells disappeared almost completely and the number of CD8⁺ T killer cells was diminished. The culture procedure used promoted mainly the expansion of T helper cells since the average CD4/CD8 ratio was 92.5. Restimulation with autologous tumour antigen resulted in clonal expansion of tumour reactive T cells as assessed by investigating the T cell receptor Vβ repertoire of sentinel node acquired lymphocytes before and after *in vitro* culture.

Before transfusion expanded T cells were functionally tested against autologous tumour antigens by measuring activation and cytokine production of the Th1 cytokine IFN-γ and the Th2 cytokine IL-4. In vitro expanded sentinel node acquired lymphocytes responded upon restimulation with tumour antigen with the production of IFN-g and no or very little IL-4 indicating that the expanded T cells were functional and Th1 responsive.

Six patients with Duke's D were treated in the study. Two patients staged as Duke's D at surgery with metastases to the liver and to the lungs and liver, respectively displayed marked regression of disease (pat 5 and 12). After transfusion of lymphocytes the first patient had total regress of liver metastases located in both lobes (which had been declared incurable by liver surgery) (figure 3) normalisation of CEA levels, disappearance of ascites and appear healthy. Patient 12 shows regress of metastases in the liver and lungs with almost a normalised CEA level at 5.9 (Normal < 4.0), disappearance of ascites and he appears clinically healthy. Patient 1 displayed a regression of the size of liver metastasis, and initially a decrease in CEA levels, disappearance of ascites and she was in excellent shape when she suddenly died (day 191), what appears to have been a lung embolus. Two Duke's D patients display stable disease without progression of metastasis or increase in CEA levels. The oldest patient no 7 in the study displayed stable disease for five months, but thereafter CEA levels started to increase and she died at age 83. No autopsy was performed. One patient was staged as Duke's C at surgery but soon developed metastases to the liver and lungs (Duke's D), but following transfusion and chemotherapy a regress of the lung and liver metastases were seen with only slightly elevated CEA levels. The patients classified as Duke's C all have normal CEA levels and appear without any signs of radiological or clinical recurrence of disease. Four of the Duke's B patients are healthy with normal CEA levels and have no signs of recurrent disease. Patient no 9 classified as Duke's B, but with an aggressive growing tumour shows signs of recurrent disease with elevated CEA levels (67) and signs of liver metastases.

To investigate the fate of transfused T cells the present inventors analysed T cell proliferation against tumour extract in peripheral blood. As mentioned before, they could not demonstrate any T cell reactivity in peripheral blood against autologous tumour antigens in any of the patients prior to transfusion. However, we were able to detect T cell proliferation against autologous tumour antigens in peripheral blood in all investigated patients up to 42 months after transfusion indicating the presence of clonally expanded circulating tumour-reactive T cells.

### Summary of patient characteristics

Below is a table of all participants in the study, sorted after Duke's classification at surgery: SD = stable disease and CR = complete response

**Participant characteristics**

| **Age/ Sex** | **Duke's Classification** | **Infused cells (x10⁶)** | **CD4/ CD8^{a}** | **IFN-γ (pg/ml)** | **Overall survival (months)** | **Response** |
|---|---|---|---|---|---|---|
| 67/M | B | 4 | 92/0.2 | ND | 31 | SD |
| 67/F | B | 8 | 15/51 | ND | 30 | SD |
| 71/M | B | 50 | 74 / 15 | 2091 | 29 | SD |
| 74/M | B | 63 | 64/22 | ND | 29 | SD |
| 66/M | B | 152 | 82/1.5 | 1411 | 27 | SD |
| 64/F | C | 110 | 64/25 | ND | 34 | SD |
| 58/F | C | 16 | 77/18 | 417 | 23 | SD |
| 61/F | D | 1 | 3.7/35 | ND | 6 | SD |
| 47/M | D | 80 | 24/16 | ND | 36 | CR |
| 54/M | D | 40 | 37/24 | ND | 36 | SD |
| 65/M | D | 270 | 82/15 | ND | 36 | CR |
| 42/F | D | 80 | 66 / 11 | ND | 33 | CR |
| 82/F | D | 40 | 98 / 0.1 | ND | 6 | SD |
| 74/M | D | 130 | 73 / 22 | 142 | 30 | CR |
| 33/M | D | 72 | 72 / 1.5 | 908 | 12 | PR |
| 66/M | D | 25 | 37 / 27 | 764 | 26 | PR |

| | | | | | | |
|---|---|---|---|---|---|---|
| ^{a}) The numbers represent the percentage of CD4 and CD8 positive cells detected with FACS. | | | | | | |

### Example 1

### Treatment of disseminated colon cancer by administering tumour-reactive T-lymphocytes and Avastin®

The patient was a 35-years old woman with rectal cancer and multiple liver metastases. She first had surgery with rectal resection with identification and harvest of sentinel nodes draining the primary tumour. Tumour-reactive T-cells were expanded according to previously described method (see herein). She received cell therapy (a transfusion of tumour-reactive T-cells) one month after surgery. After one further month she was in good clinical condition, a CT-scan showed stable disease in the liver and no other metastases. At that time she started treatment with a regular (standardized) combination of 5-fluorouracil, leucovorin, oxaliplatin and bevacizumab (Avastin®). After about one more month she received one additional transfusion of tumour-reactive T-cells based on expansion of tumour-reactive T-cells collected from her own peripheral blood. The metastases regressed continuously as seen on monthly CT-scans. After 6 months only a few necroses seemed to be left in the liver. To rule out any viable tumor the remaining dense areas in the liver were surgically resected. The histopathologic examination confirmed that there were only necrotic tissue and no tumour cells in the specimens. At the most recent follow-up visit 18 months after first operation the patient is totally free of disease according to abdominal/thoracic CT scan and normal values on tumour markers in peripheral blood.

### Example 2

### Treatment of disseminated colon cancer by administering Avastin® and tumour-reactive T-lymphocytes

Young man, aged 32 years, had diagnosis of colon cancer with intraperitoneal metastases. The patient underwent colonic resection and peritonectomy combined with intraoperative treatment with chemotherapeutic agents. Recurrence two years later first treated with 5-fluorouracil + leucovorin + oxaliplatin + bevacizumab (Avastin®). Follow-up CT-scan showed remaining progressing intraabdominal disease and elevated tumor markers in peripheral blood. At this time the patient was operated with removal of metastases involving the right colon and identification of lymph nodes draining the metastases (metinel nodes). Expansion of tumour-reactive T-cells was done based on previous method (described herein). One month after the last operation the patient had a first transfusion of tumour-reactive T-cells. The disease regressed according to CT-scans and tumour markers declined. The patient has had one more transfusion of tumour-reactive T-cells. At present he is in good condition, working full time and it is two years since the first transfusion of T-cells.

## Claims

1. A composition for use in a method of treatment of colon cancer, the composition comprising
a) tumour-reactive CD4+ helper T-lymphocytes and/or CD8+ T-lymphocytes, derived from sentinel lymph nodes and/or metinel lymph nodes; **characterized in that** it further comprises
b) one or more VEGF-inhibitors, wherein one VEGF-inhibitor is bevacizumab (Avastin^{®}),
wherein the composition is either a single composition or the composition comprises two separate preparations wherein one preparation comprises the therapeutic effective amount of tumour-reactive CD4+ helper T-lymphocytes and/or CD8+ T-lymphocytes and the second preparation comprises the therapeutic effective amount of one or more VEGF-inhibitors.

2. The composition for use in a method of treatment according to claim 1, wherein the tumour-reactive CD4+ helper and/or CD8+ T-lymphocytes are expanded T-lymphocytes and are obtained by stimulating tumour-reactive CD4+ helper and/or CD8+ T-lymphocytes with tumour-derived antigen together with at least one substance having agonistic activity towards the IL-2 receptor to promote survival of tumour-reactive CD4+ helper and/or CD8+ T-lymphocytes; and activating and promoting growth of tumour-reactive CD4+ helper and/or CD8+ T-lymphocytes at a time when the CD25 cell surface marker or IL-2R marker is downregulated on CD4+ helper and/or CD8+ T-lymphocytes.

3. The composition for use in a method of treatment according to claim 2, wherein the tumour-derived antigen is autologous.

4. The composition for use in a method of treatment according to claim 3, wherein the autologous tumour-derived antigen is tumour homogenate that is optionally denatured.

5. The composition for use in a method of treatment according to any of the preceding claims, wherein the tumour-reactive CD4+ helper T-lymphocytes and/or CD8+ T-lymphocytes comprise cancer type specific CD4+ helper T-lymphocytes and wherein at least 70% of the cancer specific CD4+ helper T-lymphocytes are of the Th1 type and 30% or less of the cancer specific CD4+ helper T-lymphocytes are of the Th2 type.

6. The composition for use in a method of treatment according to any of the preceding claims, wherein the tumour-reactive CD4+ helper T-lymphocytes and/or CD8+ T-lymphocytes comprise cancer specific T-lymphocytes of which from about 35 % to about 90 % are of the memory type.

7. The composition for use in a method of treatment according to any of claims 1-5, wherein the tumour-reactive CD4+ helper T-lymphocytes and/or CD8+ T-lymphocytes comprise cancer specific T-lymphocytes of which from about 10% to about 65% are effector T-lymphocytes.

8. The composition for use in a method of treatment according to any of claims 5-7, comprising at least 10 million or at least 20 million or at least 30 million or at least 40 million or at least 50 million or at least 100 million of the tumour-reactive CD4+ helper T-lymphocytes and/or CD8+ T-lymphocytes.

9. The composition for use in a method of treatment according to any of the preceding claims further comprising a chemotherapeutic agent selected from 5-fluorouracil, leucovorin, oxaliplatin, paclitaxel, docetaxel, fumagallin, daunorubicin, doxorubicin, epirubicin, irinotecan, topotecan, vincristine, carboplatin, cisplatin, cyclophosphamide, mitomycin C, mitoxanthrone, floxuridine, gemcitabine, methotrexate, bleomycin, etoposide, vinblastine, vindesine, vinorelbine, genistein or combinations thereof.

## Patentansprüche

1. Zusammensetzung zur Verwendung in einem Verfahren zur Behandlung von Dickdarmkrebs, wobei die Zusammensetzung umfasst
a) Tumor-reaktive CD4+ Helfer-T-Lymphozyten und/oder CD8+ T-Lymphozyten, gewonnen aus Wächterlymphknoten und/oder Metinel-Lymphknoten; **dadurch gekennzeichnet, dass** die Zusammensetzung des Weiteren enthält
b) einen oder mehrere VEGF-Inhibitoren, von denen ein VEGF-Inhibitor Bevacizumab (Avastin®) ist,
wobei die Zusammensetzung entweder eine einzige Zusammensetzung ist oder die Zusammensetzung zwei verschiedene Zubereitungen umfasst, wobei die eine Zubereitung die therapeutisch wirksame Menge an Tumor-reaktiven CD4+ Helfer-T-Lymphozyten und/oder CD8+ T-Lymphozyten enthält und die andere Zubereitung die therapeutisch wirksame Menge an einem oder mehreren VEGF-Inhibitoren enthält.

2. Zusammensetzung zur Verwendung in einem Behandlungsverfahren nach Anspruch 1, wobei die Tumor-reaktiven CD4+ Helfer- und/oder CD8+ T-Lymphozyten expandierte T-Lymphozyten sind und gewonnen werden durch die Stimulation Tumor-reaktiver CD4+ Helfer- und/oder CD8+ T-Lymphozyten mit Tumor-abgeleiteten Antigenen zusammen mit wenigstens einer weiteren Substanz, die agonistische Aktivität gegenüber dem IL-2 Rezeptor aufweist, zur Förderung des Überlebens der Tumor-reaktiven CD4+ Helfer- und/oder CD8+ T-Lymphozyten und durch Aktivierung und Förderung des Wachstums von Tumor-reaktiven CD4+ Helfer- und/oder CD8+ T-Lymphozyten zu einem Zeitpunkt, zu dem die CD25 Zelloberflächenmarker oder die IL-2R Marker auf den CD4+ Helfer- und/oder CD8+ T-Lymphozyten herabreguliert sind.

3. Zusammensetzung zur Verwendung in einem Behandlungsverfahren nach Anspruch 2, wobei das Tumor-abgeleitete Antigen autolog ist.

4. Zusammensetzung zur Verwendung in einem Behandlungsverfahren nach Anspruch 3, wobei das autologe Tumor-abgeleitete Antigen ein Tumor Homogenisat ist, das optional denaturiert ist.

5. Zusammensetzung zur Verwendung in einem Behandlungsverfahren nach einem der vorstehenden Ansprüche, wobei die Tumor-reaktiven CD4+ Helfer-T-Lymphozyten und/oder CD8+ T-Lymphozyten Krebsart-spezifische CD4+ Helfer-T-Lymphozyten umfassen und wobei mindestens 70% der Krebsart-spezifischen CD4+ Helfer-T-Lymphozyten vom Th1-Typ und 30% oder weniger der Krebsart-spezifischen CD4+ Helfer T-Lymphozyten vom Th2-Typ sind.

6. Zusammensetzung zur Verwendung in einem Behandlungsverfahren nach einem der vorstehenden Ansprüche, wobei die Tumor-reaktiven CD4+ Helfer-T-Lymphozyten und/oder CD8+ T-Lymphozyten Krebsart-spezifische T-Lymphozyten umfassen, von denen ca. 35% bis ca. 90% zu den Gedächtniszellen gehören.

7. Zusammensetzung zur Verwendung in einem Behandlungsverfahren nach einem der Ansprüche 1 bis 5, wobei die Tumor-reaktiven CD4+ Helfer-T-Lymphozyten und/oder CD8+ T-Lymphozyten Krebsart-spezifische T-Lymphozyten umfassen, von denen ca. 10% bis ca. 65% zu den Effektor-T-Lymphozyten gehören.

8. Zusammensetzung zur Verwendung in einem Behandlungsverfahren nach einem der Ansprüche 5 bis 7, umfassend mindestens 10 Millionen oder mindestens 20 Millionen oder mindestens 30 Millionen oder mindestens 40 Millionen oder mindestens 50 Millionen oder mindestens 100 Millionen der Tumor-reaktiven CD4+ Helfer-T-Lymphozyten und/oder CD8+ T-Lymphozyten.

9. Zusammensetzung zur Verwendung in einem Behandlungsverfahren nach einem der vorstehenden Ansprüche, des Weiteren umfassend einen chemotherapeutischen Wirkstoff ausgewählt aus: 5-Fluoruracil, Leucovorin, Oxaliplatin, Paclitaxel, Docetaxel, Fumagallin, Daunorubicin, Doxorubicin, Epirubicin, Irinotecan, Topotecan, Vincristin, Carboplatin, Cisplatin, Cyclophosphamid, Mitomycin C, Mitoxantron, Floxuridin, Gemcitabin, Methotrexat, Bleomycin, Etoposid, Vinblastin, Vindesin, Vinorelbin, Genistein oder Kombinationen hiervon.

## Revendications

1. Composition destinée à être utilisée dans un procédé de traitement du cancer du côlon, la composition comprenant :
a) des lymphocytes T auxiliaires CD4+ et/ou lymphocytes T CD8+ réactifs avec une tumeur, dérivés de ganglions lymphatiques sentinelles et/ou de ganglions lymphatiques métastatiques ; **caractérisée en ce qu'**elle comprend en outre
b) un ou plusieurs inhibiteurs du VEGF, où un inhibiteur du VEGF est le bévacizumab (Avastin^{®}),
où la composition est une seule composition ou la composition comprend deux préparations distinctes, où une préparation comprend la quantité thérapeutique efficace de lymphocytes T auxiliaires CD4+ et/ou lymphocytes T CD8+ réactifs avec une tumeur et la seconde préparation comprend la quantité thérapeutique efficace d'un ou de plusieurs inhibiteurs du VEGF.

2. Composition destinée à être utilisée dans un procédé de traitement selon la revendication 1, dans laquelle les lymphocytes T auxiliaires CD4+ et/ou CD8+ réactifs avec une tumeur sont des lymphocytes T ayant subi une expansion et sont obtenus en stimulant des lymphocytes T auxiliaires CD4+ et/ou CD8+ réactifs avec une tumeur avec un antigène dérivé d'une tumeur et avec au moins une substance ayant une activité agoniste envers le récepteur de l'IL-2 afin de favoriser la survie des lymphocytes T auxiliaires CD4+ et/ou CD8+ réactifs avec une tumeur ; et en activant et en favorisant la croissance des lymphocytes T auxiliaires CD4+ et/ou CD8+ réactifs avec une tumeur à un moment où le marqueur de surface cellulaire CD25 ou le marqueur IL-2R est négativement régulé sur les lymphocytes T auxiliaires CD4+ et/ou CD8+.

3. Composition destinée à être utilisée dans un procédé de traitement selon la revendication 2, dans laquelle l'antigène dérivé d'une tumeur est autologue.

4. Composition destinée à être utilisée dans un procédé de traitement selon la revendication 3, dans laquelle l'antigène dérivé d'une tumeur autologue est un homogénat tumoral qui est facultativement dénaturé.

5. Composition destinée à être utilisée dans un procédé de traitement selon l'une quelconque des revendications précédentes, dans laquelle les lymphocytes T auxiliaires CD4+ et/ou lymphocytes T CD8+ réactifs avec une tumeur comprennent des lymphocytes T auxiliaires CD4+ spécifiques à un type de cancer et dans laquelle au moins 70 % des lymphocytes T auxiliaires CD4+ spécifiques à un cancer sont du type Th1 et 30 % ou moins des lymphocytes T auxiliaires CD4+ spécifiques à un cancer sont du type Th2.

6. Composition destinée à être utilisée dans un procédé de traitement selon l'une quelconque des revendications précédentes, dans laquelle les lymphocytes T auxiliaires CD4+ et/ou lymphocytes T CD8+ réactifs avec une tumeur comprennent des lymphocytes T spécifiques à un cancer parmi lesquels entre environ 35 % et environ 90 % sont du type mémoire.

7. Composition destinée à être utilisée dans un procédé de traitement selon l'une quelconque des revendications 1-5, dans laquelle les lymphocytes T auxiliaires CD4+ et/ou lymphocytes T CD8+ réactifs avec une tumeur comprennent des lymphocytes T spécifiques à un cancer parmi lesquels entre environ 10 % et environ 65 % sont des lymphocytes T effecteurs.

8. Composition destinée à être utilisée dans un procédé de traitement selon l'une quelconque des revendications 5 à 7, comprenant au moins 10 millions ou au moins 20 millions ou au moins 30 millions ou au moins 40 millions ou au moins 50 millions ou au moins 100 millions des lymphocytes T auxiliaires CD4+ et/ou lymphocytes T CD8+ réactifs avec une tumeur.

9. Composition destinée à être utilisée dans un procédé de traitement selon l'une quelconque des revendications précédentes, comprenant en outre un agent de chimiothérapie sélectionné parmi le 5-fluorouracile, la leucovorine, l'oxaliplatine, le paclitaxel, le docétaxel, la fumagalline, la daunorubicine, la doxorubicine, l'épirubicine, l'irinotécan, le topotécan, la vincristine, le carboplatine, le cisplatine, le cyclophosphamide, la mitomycine C, la mitoxantrone, la floxuridine, la gemcitabine, le méthotrexate, la bléomycine, l'étoposide, la vinblastine, la vindésine, la vinorelbine, la génistéine ou des combinaisons de ceux-ci.
